# EUROPÄISCHE PATENTSCHRIFT

(11) **EP 1 727 820 B1**
(45) Veröffentlichungstag und Bekanntmachung des Hinweises auf die Patenterteilung: **14.09.2011**
(21) Anmeldenummer: 04797640.2
(22) Anmeldetag: 05.11.2004
(51) Int. Cl.: C07D 487/04, C07D 231/38, C07D 249/14, A61K 31/519, C07D 471/14, A61P 35/00

(54) **AMINDERIVATE MIT TYROSINKINASE HEMMENDER WIRKUNG**
AMINE DERIVATIVES WITH TYROSINKINASE INHIBITING ACTIVITY
DERIVES D'AMINE AYANT UNE ACTIVITE EN TANT QU'INHIBITEURS DE LA TYROSINE KINASE

(30) Priorität: 04.12.2003 DE 10356579
(43) Veröffentlichungstag der Anmeldung: 06.12.2006
(73) Patentinhaber: Merck Patent GmbH, 64293 Darmstadt (DE)
(72) Erfinder: SCHIEMANN, Kai, 64342 Seeheim-Jugenheim (DE); HÖLZEMANN, Günter, 64342 Seeheim-Jugenheim (DE); RAUTENBERG, Wilfried, 64354 Reinheim (DE)
(86) Internationale Anmeldenummer: PCT/EP2004/012523
(87) Internationale Veröffentlichungsnummer: WO 2005/054246

(56) Entgegenhaltungen:
- WO-A-02/50079
- WO-A-96/31510
- WO-A-98/54093
- WO-A-02/064211
- US-A- 4 576 943
- US-A- 4 582 833
- REITER J ET AL: "On triazoles. XXXV. The reaction of 5-amino-1,2,4-triazoles with di- and triketones" JOURNAL OF HETEROCYCLIC CHEMISTRY 1995 UNITED STATES, Bd. 32, Nr. 2, 1995, Seiten 407-417, XP002322758 ISSN: 0022-152X
- OKABE T ET AL: "DI ALKYL BI CYCLIC HETERO CYCLES WITH A BRIDGEHEAD NITROGEN AS PURINE ANALOGS POSSESSING SIGNIFICANT CARDIAC INOTROPIC ACTIVITY" JOURNAL OF HETEROCYCLIC CHEMISTRY, Bd. 20, Nr. 3, 1983, Seiten 735-751, XP002322759 ISSN: 0022-152X

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten.

Im einzelnen betrifft die vorliegende Erfindung einzhne Verbindungen gamäß Anspruch 1 umfaßt von der Formel I, die die Signaltransduktion der Tyrosinkinasen hemmen, regulieren und/oder modulieren, Zusammensetzungen, die diese Verbindungen enthalten, sowie Verfahren zu ihrer Verwendung zur Behandlung von tyrosinkinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Bei den Tyrosinkinasen handelt es sich um eine Klasse von Enyzmen mit mindestens 400 Mitgliedern, die die Übertragung des endständigen Phosphats des Adenosintriphosphats (gamma-Phosphat) auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genauen Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.
Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen. (siehe Reviews von Schlessinger und Ullrich, Neuron 9, 383-391 (1992) und 1-20 (1992)).
Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung HER-Unterfamilie trägt, besteht aus EGFR, HER2, HER3 und HER4. Zu den Liganden dieser Rezeptor-Unterfamilie zählen der Epithel-Wachstumsfaktor, TGF-α, Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR), der fötalen Leberkinase-1 (FLK-1), der fötalen Leberkinase-4 (FLK-4) und der fms-Tyrosinkinase-1 (flt-1) besteht. Die PDGF- und FLK-Familie werden üblicherweise aufgrund der zwischen den beiden Gruppen bestehenden Ähnlichkeiten gemeinsam diskutiert. Für eine genaue Diskussion der Rezeptor-Tyrosinkinasen siehe die Arbeit von Plowman et al., DN & P 7(6):334-339, 1994, die hiermit durch Bezugnahme aufgenommen wird.
Zu den RTKs (Rezeptor-Tyrosin-Kinasen) gehören auch TIE2 und seine Liganden Angiopoietin 1 und 2. Es werden mittlerweile immer mehr Homologe dieser Liganden gefunden, deren Wirkung im Einzelnen noch nicht klar nachgewiesen wurde. Als Homologes von TIE2 ist TIE1 bekannt. Die TIE RTKs werden selektiv auf Endothelzellen exprimiert und finden ihre Aufgabe bei Prozessen der Angiogenese und Maturierung der Blutgefäße. Dadurch können sie insbesondere bei Erkrankungen des Gefäßsystems und bei Pathologien, in denen Gefäße genutzt oder gar umgebildet werden, ein wertvolles Ziel sein. Ausser der Verhinderung der Gefäßneubildung und Maturierung kann auch die Stimulation von Gefäßneubildung ein wertvolles Ziel für Wirkstoffe sein. Bezug genommen wird auf Übersichtsarbeiten zur Angiogenese, Tumorentwicklung und Kinase Signalgebung von
G. Breier Placenta (2000) 21, Suppl A, Trophoblasr Res 14, S11-S15
F. Bussolino et al. TIBS 22, 251 -256 (1997)
G. Bergers & L.E. Benjamin Nature Rev Cancer 3, 401-410 (2003)
P. Blume-Jensen &. Hunter Nature 411, 355-365 (2001)
M. Ramsauer & P. D'Amore J. Clin. INvest. 110, 1615-1617 (2002)
S. Tsigkos et al. Expert Opin. Investig. Drugs 12, 933-941 (2003)

Beispiele für Kinase-Inhibitoren, die bereits in der Krebstherapie getestet werden, können L.K. Shawyer et al. Cancer Cell 1, 117-123(2002) und D. Fabbro & C. Garcia-Echeverria Current Opin. Drug Discovery & Development 5, 701-712 (2002) entnommen werden.

Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Rezeptoren unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen Oncogene, 8:2025-2031 (1993), die hiermit durch Bezugnahme aufgenommen wird.
Sowohl die Rezeptor-Tyrosinkinasen als auch die zytosolischen Tyrosinkinasen sind an Signalübertragungswegen der Zelle, die zu verschiedenen Leidenszuständen führen, darunter Krebs, Schuppenflechte und Hyperimmunreaktionen, beteiligt.
Es wurde vorgeschlagen, dass verschiedene Rezeptor-Tyrosinkinasen sowie die an sie bindenden Wachstumsfaktoren eine Rolle bei den Angiogenese spielen, obwohl einige die Angiogenese indirekt fördern könnten (Mustonen und Alitalo, J. Cell Biol. 129:895-898, 1995). Eine dieser Rezeptor-Tyrosinkinasen ist die fötale Leberkinase 1, auch FLK-1 genannt. Das menschliche Analog der FLK-1 ist der kinase-insertdomänenhaltige Rezeptor KDR, der auch unter der Bezeichnung Gefäßendothelzellenwachstumsfaktorrezeptor 2 bzw. VEGFR-2 bekannt ist, da er VEGF hochaffin bindet. Schließlich wurde die Maus-Version dieses Rezeptors auch ebenfalls NYK genannt (Oelrichs et al., Oncogene 8(1):11-15, 1993). VEGF und KDR stellen ein Ligand-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.
Der Vorgang der Angiogenese stellt die Entwicklung von neuen Blutgefäßen, in der Regel Kapillaren, aus dem bereits bestehenden Gefäßsystem dar. Angiogenese ist so definiert, dass sie (i) die Aktivierung von Endothelzellen; (ii) eine erhöhte Gefäßpermeabilität; (iii) anschließende Auflösung der Basalmembran und Extravasation von Plasmabestandteilen, die zur Bildung einer vorläufigen extrazellulären Fibringel-Matrix führen; (iv) die Proliferation und Mobilisierung von Endothelzellen; (v) die Reorganisation von mobilisierten Endothelzellen zur Bildung funktioneller Kapillaren; (vi) die Bildung des Kapillarschenkels und (vii) die Ablagerung einer Basalmembran sowie die Rekrutierung perivaskulärer Zellen zu neu gebildeten Gefäßen umfasst.

Eine normale Angiogenese wird während des Gewebswachstums von der Embryonalentwicklung bis zur Reifung aktiviert und tritt dann während des Erwachsenenlebens in einen Zeitraum relativer Ruhe ein.

Eine normale Angiogenese wird auch während der Wundheilung und in bestimmten Stadien des weiblichen Reproduktionszyklus' aktiviert. Unangemessene oder pathologische Angiogenese wurde mit verschiedenen Erkrankungszuständen in Verbindung gebracht, einschließlich verschiedener Retinopathien; ischämischer Erkrankung; Atherosklerose; chronischer entzündlicher Störungen; rheumatoider Arthritis und Krebs. Die Rolle der Angiogenese bei Erkrankungszuständen ist zum Beispiel in Fan et al., Trends in Pharmacol Sci. 16:54 66; Shawver et al., DOT Bd. 2, Nr. 2 Februar 1997; Folkmann, 1995, Nature Medicine 1:27-31 erläutert.

Bei Krebs wurde gezeigt, dass das Wachstum solider Tumore Angiogenese-abhängig ist. (Siehe Folkmann, J., J. Nat'l. Cancer Inst., 1990, 82, 4-6). Folglich ist die Ansteuerung pro-angiogenetischer Wege eine Strategie, die weitverbreitet verfolgt wird, um neue Therapeutika auf diesen Gebieten eines großen, unerfüllten medizinischen Bedarfs bereitzustellen.

Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, Cytokine & Growth Factor Reviews 7:259-270, 1996). Der VEGF bindet den hochaffinen transmembranösen Tyrosinkinaserezepzor KDR und die verwandte fms-Tyrosinkinase-1, auch unter der Bezeichnung Flt-1 oder Gefäßendothelzellenwachstumsfaktorrezeptor 1 (VEGFR-1) bekannt. Aus Zellkultur- und Gen- Knockout-Versuchen geht hervor, dass jeder Rezeptor zu unterschiedlichen Aspekten der Angiogenese beiträgt. Der KDR führt die mitogene Funktion des VEGF herbei, während Flt-1 nichtmitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 362, S. 841- 844, 1993). Drei PTK (Protein-Tyrosinkinase)-Rezeptoren für VEGFR sind identifiziert worden : VEGFR-1 (Flt-1); VEGRF-2 (Flk-1 oder KDR) und VEGFR-3 (Flt-4). Von besonderem Interesse ist VEGFR-2.

Feste Tumore können daher mit Tyrosinkinasehemmern behandelt werden, da diese Tumore für die Bildung der zur Unterstützung ihres Wachstums erforderlichen Blutgefäße auf Angiogenese angewiesen sind. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom. Zu weiteren Beispielen zählen Karzinome, bei denen eine Überexpression oder Aktivierung von Rafaktivierenden Onkogenen (z.B. K-ras, erb-B) beobachtet wird. Zu diesen Karzinomen zählen Bauchspeicheldrüsen- und Brustkarzinom. Hemmstoffe dieser Tyrosinkinasen eignen sich daher zur Vorbeugung und Behandlung von proliferativen Krankheiten, die durch diese Enzyme bedingt sind.
Die angiogene Aktivität des VEGF ist nicht auf Tumore beschränkt. Der VEGF ist für die bei diabetischer Retinopathie in bzw, in der Nähe der Retina produzierte angiogene Aktivität verantwortlich. Dieses Gefäßwachstum in der Retina führt zu geschwächter Sehkraft und schließlich Erblindung. Die VEGF-mRNA- und -protein-Spiegel im Auge werden durch Leiden wie Netzhautvenenokklusion beim Primaten sowie verringertem pO₂-Spiegel bei der Maus, die zu Gefäßneubildung führen, erhöht. Intraokular injizierte monoklonale Anti-VEGF-Antikörper, oder VEGF-Rezeptor-Immunkonjugate, hemmen sowohl im Primaten- als auch im Nagetiermodell die Gefäßneubildung im Auge. Unabhängig vom Grund der Induktion des VEGF bei der diabetischen Retinopathie des Menschen, eignet sich die Hemmung des Augen-VEGF zur Behandlung dieser Krankheit.

Die VEGF-Expression ist auch in hypoxischen Regionen von tierischen und menschlichen Tumoren neben Nekrosezonen stark erhöht. Der VEGF wird außerdem durch die Expression der Onkogene ras, raf, src und p53-Mutante (die alle bei der Bekämpfung von Krebs von Bedeutung sind) hinaufreguliert. Monoklonale Anti-VEGF-Antikörper hemmen bei der Nacktmaus das Wachstum menschlicher Tumore. Obwohl die gleichen Tumorzellen in Kultur weiterhin VEGF exprimieren, verringern die Antikörper ihre Zellteilungsrate nicht. So wirkt der aus Tumoren stammende VEGF nicht als autokriner mitogener Faktor. Der VEGF trägt daher in vivo dadurch zum Tumorwachstum bei, dass er durch seine parakrine Gefäßendothelzellen-Chemotaxis- und -Mitogeneseaktivität die Angiogenese fördert. Diese monoklonalen Antikörper hemmen auch das Wachstum von typischerweise weniger stark vaskularisierten Human-Kolonkarzinomen bei thymuslosen Mäusen und verringern die Anzahl der aus inokulierten Zellen entstehenden Tumore.
Die Expression eines VEGF-bindenden Konstrukts von Flk-1, Flt-1, dem zur Entfernung der zytoplasmatischen Tyrosinkinasedomänen, jedoch unter Beibehaltung eines Membranankers, verkürzten Maus-KDR-Rezeptorhomologs, in Viren stoppt praktisch das Wachstum eines transplantierbaren Glioblastoms bei der Maus, vermutlich aufgrund des dominant-negativen Mechanismus der Heterodimerbildung mit transmembranösen Endothelzellen-VEGF-Rezeptoren. Embryostammzellen, die in der Nacktmaus üblicherweise in Form von festen Tumoren wachsen, bilden bei Knock-out aller beider VEGF-Allele keine nachweisbaren Tumore. Aus diesen Daten gemeinsam geht die Rolle des VEGF beim Wachstum fester Tumore hervor. Die Hemmung von KDR bzw. Flt-1 ist an der pathologischen Angiogenese beteiligt, und diese Rezeptoren eignen sich zur Behandlung von Krankheiten, bei denen Angiogenese einen Teil der Gesamtpathologie, z.B. Entzündung, diabetische Retina-Vaskularisierung sowie verschiedene Formen von Krebs, darstellt, da bekannt ist, dass das Tumorwachstum angiogeneseabhängig ist (Weidner et al., N. Engl. J. Med., 324, S. 1-8,1991).

Bei Angiopoietin 1 (Ang1), einem Liganden für die endothelspezifische Rezeptor-Tyrosinkinase TIE-2, handelt es sich um einen neuen angiogenen Faktor (Davis et al, Cell, 1996, 87:1161-1169; Partanen et al, Mol. Cell Biol., 12:1698-1707 (1992); US-Patent Nr. 5,521,073; 5,879,672; 5,877,020; und 6,030,831). Das Akronym TIE steht für "Tyrosinkinase mit Ig- und EGF-Homologiedomänen". TIE wird zur Identifizierung einer Klasse von Rezeptor-Tyrosinkinasen verwendet, die ausschließlich in Gefäßendothelzellen und frühen hämopoietischen Zellen exprimiert werden. TIE-Rezeptorkinasen sind typischerweise durch das Vorhandensein einer EGF-ähnlichen Domäne und einer Immunglobulin (IG)-ähnlichen Domäne charakterisiert, die aus extrazellulären Faltungseinheiten, die durch Disulfidbrückenbindungen zwischen den Ketten stabilisiert sind, besteht (Partanen et al Curr. Topics Microbiol. Immunol., 1999, 237:159-172). Im Gegensatz zu VEGF, der seine Funktion während der frühen Stadien in der Gefäßentwicklung ausübt, wirken Ang1 und sein Rezeptor TIE-2 während der späteren Stadien in der Gefäßentwicklung, d.h. während der Gefäßumbildung (Umbildung bezieht sich auf die Bildung eines Gefäßlumens) und Reifung (Yancopoulos et al, Cell, 1998, 93:661-664; Peters, K.G., Circ. Res., 1998, 83(3):342-3; Suri et al, Cell 87, 1171-1180 (1996))._{.}

Demzufolge würde man erwarten, daß eine Hemmung von TIE-2 die Umbildung und Reifung eines durch Angiogenese initiierten neuen Gefäßsystems und dadurch den Angiogeneseprozeß unterbrechen sollte. Weiterhin würde eine Hemmung an der Kinasedomäne-Bindungsstelle von VEGFR-2 die Phosphorylierung von Tyrosinresten blockieren und dazu dienen, die Initiation der Angiogenese zu unterbrechen. Daher darf man annehmen, daß die Hemmung von TIE-2 und/oder VEGFR-2 die Tumorangiogenese verhindern und dazu dienen sollte, das Tumorwachstum zu verlangsamen oder vollständig zu beseitigen. Dementsprechend könnte man eine Behandlung von Krebs und anderen mit unangemessener Angiogenese einhergehenden Erkrankungen bereitstellen.

Wie hierin besprochen, sind die beschriebenen Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind.

Die vorliegende Erfindung richtet sich auf Verfahren zur Regulation, Modulation oder Hemmung der TIE-2 zur Vorbeugung und/oder Behandlung von Erkrankungen im Zusammenhang mit unregulierter oder gestörter TIE-2-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I gemäß Anspruch 1 zur Isolierung und zur Untersuchung der Aktivität oder Expression von TIE-2 verwendet werden. Außerdem eigenen sie sich insbesondere zur Verwendung in diagnostischen Verfahren zu Erkrankungen im Zusammenhang mit unregulierter oder gestörter TIE-2-Aktivität.

Die vorliegende Erfindung richtet sich weiterhin auf Verfahren zur Regulation, Modulation oder Hemmung des VEGFR-2 zur Vorbeugung und/oder Behandlung von Erkrankungen im Zusammenhang mit unregulierter oder gestörter VEGFR-2-Aktivität.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Proteinkinase PKB (auch als AKT und RAC-PK bekannt) ist ein Mitglied der AKT/PKB-Familie der Serin-/Threonin-Kinasen, und es wurde gezeigt, dass sie an einer diversen Reihe von Signalwegen bei der humanen Malignität beteiligt ist (Nicholson et al., Cell. Signal., 2002, 14, 381-395). PKB, wie auch andere Mitglieder der AKT/PKB-Familie, ist im Cytosol nicht stimulierter Zellen lokalisiert und transloziert nach Stimulation an die Zellmembran. PKB-Translokation kann durch mehrere Liganden, einschließlich des aus Thrombozyten stammenden Wachstumsfaktors, epidermalen Wachstumfaktors, basischen Fibrobiasten-Wachstumsfaktors, Zellstress, wie zum Beispiel Hitzeschock und Hyperosmolarität und auch Insulin, aktiviert werden (Bos, Trends Biochem. Sci., 1995, 20, 441-442), und andere Studien haben gezeigt, dass diese Aktivierung über PI3-Kinase abläuft, die Wortmannin-empfindlich ist (Franke et al., Science, 1997, 275, 665-668). Sobald PKB an der Plasmamembran lokalisiert ist, wurde gezeigt, dass sie mehrere Funktionen in der Zelle vermittelt, einschließlich Apoptose, der metabolischen Effekte von Insulin, Induktion von Differenzierung und/oder Proliferation, Proteinsynthese und Stressantworten (Alessi und Cohen, Curr. Opin. Genet. Dev., 1998, 8, 55-62; Downward, Curr. Opin. Cell Biol., 1998, 10, 262-267).

PKB wurde 1991 von drei Gruppen unabhängig geklont (Bellacosa et al., Science, 1991, 254, 274-277; Coffer und Woodgett, Eur. J. Biochem., 1991, 201, 475-481; Jones et al., Cell Regul., 1991, 2, 1001-1009), ihr Zusammenhang mit dem primären humanen Magenkarzinom wurde jedoch bereits 1987 erkannt (Staal et al., Proc. Natl. Acad. Sci. U S A, 1987, 84, 5034-5037). Sequenzierung von PKBα ließ in den Kinasedomänen Homologie zu den PKA- (ca. 68 %) und PKC-Isozymen (ca. 73 %) erkennen (Jones et al., Proc. Natl. Acad. Sci. U.S.A., 1991, 88, 4171-5), eine Tatsache, die zu ihrer Umbenennung in PKB führte. Es gibt drei zelluläre PKB-Isoformen und zwei Splice-Varianten (PKBα, β, γ, β₁, γ₁; Brazil et al. Trends in Bio Sci, 2001, 26, 657-663). Es wurde gefunden, dass PKBα bei Magenadenokarzinomen und einer Brustkrebs-Zelllinie amplifiziert oder überexprimiert wird (Staal et al., Proc. Natl. Acad. Sci. U.S.A., 1987, 84, 5034-7; Jones et al., Cell Regul., 1991, 2, 1001-9). PKBβ wird bei 3 % des Brust- (Bellacosa et al., Int. J. Cancer, 1995 64, 280-5), 12 % des Pankreas- (Cheng et al., Proc. Natl. Acad. Sci. U.S.A., 1996, 93, 3636-41) und 15 % des Eierstockkrebses amplifiziert oder überexprimiert (Bellacosa et al., Int. J. Cancer, 1995, 64, 280-5; Cheng et al., Proc. Natl. Acad. Sci. U.S.A., 1992,89,9267-71).

PKBγ wird bei Estrogenrezeptor-defizientem Brustkrebs und bei Androgenunabhängigen Prostata-Zelllinien überexprimiert (Nakatani et al., J. Biol. Chem. 1999, 274, 21528-32).

Es wurde vorgeschlagen, dass PKB ein an der chromosomalen Umordnung an der Chromosomenbande 14q32 beteiligtes Gen ist. Von diesem Locus ist bekannt, dass er bei humanen T-Zellmalignitäten, wie zum Beispiel bei prolymphozytischen Leukämien und Leukämien gemischter Abstammung im Kindersalter der Umordnung unterliegt (Staal et al., Genomics, 1988, 2, 96-98).

PKB spielt auch eine Rolle bei der Verhinderung des "programmierten Zelltodes" oder der Apoptose durch inhibitorische Phosphorylierung von ASK-1, Bad, Caspase9 und FKHR (Übersicht siehe Nicholson et al., Cell Signaling 2001, 14, 281-395). Es wurde nachgewiesen, dass PKB ein Überlebenssignal (Übersicht siehe Lawlor et al., J. of Cell Science 2001, 114, 2903-2910) für Zellen liefert, um sie vor einer Anzahl von Agenzien, einschließlich UV-Strahlung (Dudek et al., Science, 1997, 275, 661-665), Entzug von IGF1 aus neuronalen Zellen, Ablösung von der extrazellulären Matrix, Stress und Hitzeschock zu schützen (Alessi und Cohen, Curr. Opin. Genet. Dev., 1998, 8, 55-62).

Die dual-spezifische Phosphatase PTEN (Phosphatase and Tensin homologue deleted on Chromosome Ten [Phosphatase und Tensin homolog deletiert an Chromosom zehn]) erhöht den Ptdlns(3, 4, 5)P₃-Spiegel in der Zelle durch Dephosphorylierung von Ptdlns(3, 4, 5)P₃. Ptdlns(3, 4, 5)P₃ bindet an die PH-Domäne (Pleckstrin-Homologie- Domäne) von PKB. Diese Bindung stellt einen wesentlichen Schritt für die Membran-Translokation und Aktivierung von PKB dar. PTEN ist ein in einer großen Fraktion von Glioblastom- und Melanom-Zelllinien, fortgeschrittenen Prostatakarzinomen und endometrialen Karzinomen mutiertes Tumor-Suppressor-Gen. Es ist darüber hinaus bei >80 % der Patienten mit erblichen Leiden, wie zum Beispiel Cowden-Syndrom, Lhermitte-Duclos-Syndrom und Bannayan-Zonana-Syndrom deletiert. Die Patienten weisen mehrere ähnliche Merkmale auf, einschließlich multipler gutartiger Tumoren

(Harmatomen) und einer erhöhten Anfälligkeit für Brust- und Schilddrüsenmalignitäten (Di Cristofano et al. Cell, 2000, 100, 387-390).

Von heterozygoten PTEN^{+/-}-Mäusen (heterozygote PTEN^{-/-}-Mäuse sind nicht lebensfähig) hergeleitete Zelllinien weisen erhöhte Ptdlns(3, 4, 5)P₃-Spiegel auf, die mit erhöhter PKB-Aktivität, mit einer gleichzeitig verminderten Sensitivität gegen Apoptose einhergehen (Di Christofano et al. Nat. Genet. 1998, 19, 348-355; Stambolic et al., Cell, 1998, 95, 29-39, Myers et al., Proc. Natl. Acad. Si. U.S.A., 1998, 96 13513-13518).

PKB ist auch zur Promotion der Zellzyklus-Progression durch Inhibition des p21-Zellzyklus-Inhibitors fähig (Zhou et al.; Nat. Cell Biol., 2002,3, 245-252).

Diese Befunde könnten die Überexperession von PKB erklären, die in Krebszellen beobachtet wird, die präferenzielles Überleben und Proliferation der Karzinome durch Verhindern der normalen Progression zur Apoptose ermöglicht.
Zur Zeit gibt es keine bekannten Therapeutika, welche die PKB-Aktivität wirksam inhibieren. Folglich besteht noch immer ein seit langem wahrgenommener Bedarf an zusätzlichen Mitteln, die als Chemotherapeutika zur wirksamen Inhibition der PKB-Funktion zur Aktivierung von proapoptotischen Proteinen bei allen Krebsarten fähig sind.

Die Synthese von kleinen Verbindungen, die die Signaltransduktion der Tyrosinkinasen spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Es wurde überraschend gefunden, daß die erfindungsgemäßen Verbindungen mit Signalwegen, besonders mit den hierin beschriebenen Signalwegen interagieren können. Die erfindungsgemäßen Verbindungen zeigen bevorzugt eine vorteilhafte biologische Aktivität, die in auf Enzymen basierenden Assays, zum Beispiel Assays wie hierin beschrieben, leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind. Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren.
Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung. In einer Vergleichsmessung wurde weiterhin gefunden, daß die Verbindungen der Formel I als PKB-Inhibitoren wirken. Diese Wirkung kann zum Beispiel durch ein Verfahren nachgewiesen werden, das von Alessi et al. EMBO L. 1996, 15, 6541-6551 beschrieben wird.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene AssaySysteme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die erfindungsgemäßen Verbindungen eignen sich auch als p38 Kinase-Inhibitoren.
Heteroarylharnstoffe, die p38 Kinase inhibieren sind in der WO 02/85859 beschrieben.

### STAND DER TECHNIK

Triazolo[1,5-a]pyrimidin-2-yl-aminderivate sind in WO 02/064211 als Thrombininhibitoren beschrieben.

### ZUSAMMENFASSUNG DER ERFINDUNG

Die Erfindung betrifft Verbindungen gemäß Anspruch 1 umfaßt von der Formel I 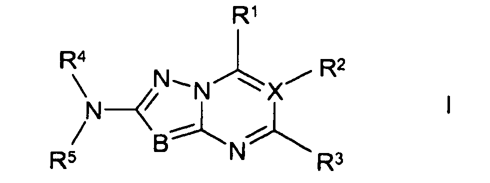 worin
- X: C oder N,
- B: N, CH oder C-CN,
- R¹: H, A, OH, NH₂, -(CH₂)ₘ-Ar oder -(CH₂)ₘ-Het²,
- R²: wenn X = N fehlt oder wenn X = C H, A, Hal, CN, -(CH₂)ₚ-Ar, -(CH₂)ₚ-COOH, -(CH₂)ₚ-COOA, -(CH₂)ₚ-Het³, -(CH₂)ₚ-NH₂, SO₂A, CHO oder COA,
- R³: H, A, -S-A, -(CH₂)ₚ-Ar, -(CH₂)ₚ-Het, NH-(CH₂)ₚ-Ar, NH-(CH₂)ₚ-Het, NH₂, NHA, NA₂, NH-Alkylen-NH₂, NH-Alkylen-NHA, NH-Alkylen-NA₂ oder NA-Alkylen-NA₂,
- R⁴: -(CH₂)ₛ-(Ar¹)ₙ-Y-R⁶,
- R⁵: H oder CH₃,

- R⁴ und R⁵: zusammen auch 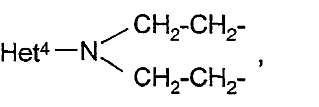
- R⁶: Het⁴, -(CH₂)ᵣ-NH₂, -(CH₂)ᵣ-NHA oder -(CH₂)ᵣ-NA₂,
- Y: O, S, (CH₂)_{q} oder NH,
- Ar: unsubstituiertes oder ein-, zwei- oder dreifach durch Hal, A, OH, OA, NH₂, NO₂, CN, COOH, COOA, CONH₂, NHCOA, NHCONH₂, NHSO₂A CHO, COA, SO₂NH₂, SO₂A, -CH₂- COOH oder -OCH₂-COOH substituiertes Phenyl, Naphthyl oder Biphenyl,
- Ar¹: Phenylen oder Piperazin-diyl,
- Het: einen ein- oder zweikernigen gesättigten, ungesättigten oder aromatischen Heterocyclus mit 1 bis 4 N-, O- und/oder S- Atomen, der unsubstituiert oder ein-, zwei- oder dreifach durch Hal, A, NHA, NA₂, OA, COOA, CN, -(CH₂)ₚ-Ar, -(CH₂)ₜ-OH, -(CH₂)ₚ-Het¹ oder Carbonylsauerstoff (=O) substituiert sein kann,
- Het¹: einen ein- oder zweikernigen gesättigten, ungesättigten oder aromatischen Heterocyclus mit 1 bis 4 N-, O- und/oder S- Atomen, der unsubstituiert oder ein- oder zweifach durch A oder Carbonylsauerstoff (=O) substituiert sein kann,
- Het²: einen einkernigen aromatischen Heterocyclus mit 1 bis 3 N-, O- und/oder S-Atomen, der unsubstituiert oder ein- oder zweifach durch A substituiert sein kann,
- Het³: einen einkernigen gesättigten oder aromatischen Heterocyclus mit 1 bis 3 N-, O- und/oder S-Atomen, der unsubstituiert oder ein- oder zweifach durch A substituiert sein kann,
- Het⁴: einen ein- oder zweikernigen gesättigten, ungesättigten oder aromatischen Heterocyclus mit 1 bis 4 N-, O- und/oder S- Atomen, der unsubstituiert oder ein-, zwei- oder dreifach durch Hal, A, CONH₂, CONHA, CONA₂ oder Ar² substituiert sein kann,
- Ar²: unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂ oder SO₂A substituiertes Phenyl,
- R⁷, R⁸, R⁹, R¹⁰: jeweils unabhängig voneinander H, A oder -(CH₂)ₚ-Ar,
- A: Alkyl mit 1 bis 10 C-Atomen, wobei auch 1-7 H-Atome durch F und/oder Chlor ersetzt sein können,
- m: 0, 1, 2, 3 oder 4,
- n: 0 oder 1,
- p: 0, 1, 2, 3 oder 4,
- q: 0, 1, 2, 3 oder 4,
- r: 0, 1, 2, 3 oder 4,
- s: 0, 1, 2, 3 oder 4,
- Hal: F, Cl, Br oder I,
bedeuten,
und, wenn X = C
R¹ und R² zusammen auch -(CH₂)₄- oder
R² und R³ zusammen auch -(CHR⁷-NR⁸-CHR⁹-CHR¹⁰)-
bedeuten können,
und, wenn Ar¹ Piperazin-diyl bedeutet, R⁶ auch H oder Alkyl mit 1-6 C-Atomen bedeuten kann,
sowie ihre pharmazeutisch verwendbaren Solvate, Tautomere, Salze und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat: verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I gamäß Anspruch 1 und ihre Salze sowie ein Verfahren zur Herstellung von Verbindungen der Formel I gamäß Anspruch 1 sowie ihrer pharmazeutisch verwendbaren Salze, Solvate und Stereoisomeren, dadurch gekennzeichnet, daß man
a) zur Herstellung von Verbindungen der Formel I gemäß Anspruch 1,
   worin X C bedeutet, eine Verbindung der Formel II 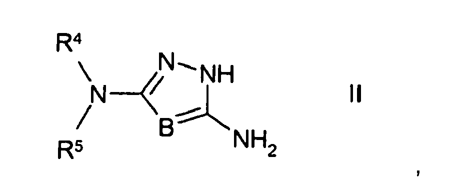 worin R⁴, R⁵ und B die in Anspruch 1 entsprechenden Bedeutungen haben,
   i) mit einer Verbindung der Formel IIIa 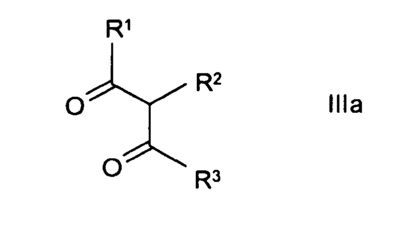 worin R¹ OA und
      R² und R³ die in Anspruch 1 entsprechenden Bedeutungen haben, umsetzt,
      oder
   ii) mit einer Verbindung der Formel IIIb 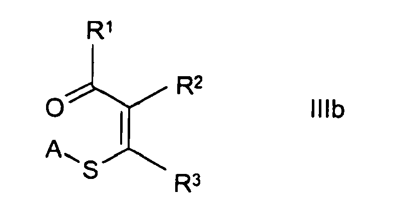 worin R¹ R² und R³ die in Anspruch 1 entsprechenden Bedeutungen haben, und A Alkyl mit 1, 2, 3 oder 4 C-Atomen bedeutet,
      umsetzt,
      oder
   iii) mit einer Verbindung der Formel IIIc 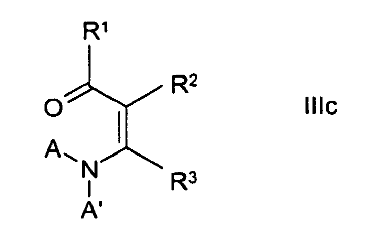 worin
      R¹ neben den in Anspruch 1 entsprechenden Bedeutungen auch OA bedeutet,
      R² und R³ die in Anspruch 1 entsprechenden Bedeutungen haben,
      und A, A' jeweils unabhängig voneinander Alkyl mit 1, 2, 3 oder 4 C-Atomen bedeuten,
      oder A und A' zusammen auch eine Butylen- oder Pentylenkette bilden können,
      umsetzt,
      oder
b) zur Herstellung von Verbindungen der Formel I,
   worin X N und R¹ NH₂ bedeuten,
   eine Verbindung der Formel II mit einer Verbindung der Formel IIId 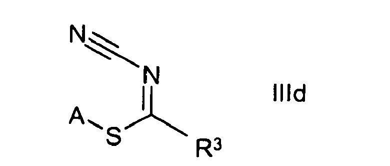 worin R³ die in Anspruch 1 entsprechende Bedeutung hat, und A Alkyl mit 1, 2, 3 oder 4 C-Atomen bedeutet,
   umsetzt,
   oder
c) zur Herstellung von Verbindungen der Formel I gemäß Anspruch 1, worin
   - X: N,
   - R¹: H, A, -(CH₂)ₘ-Ar oder -(CH₂)ₘ-Het²,
   - R³: -S-A
   bedeuten,
   eine Verbindung der Formel II mit einer Verbindung der Formel IIIe 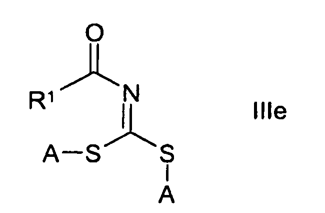 worin
   - R¹: H, A, -(CH₂)ₘ-Ar oder -(CH₂)ₘ-Het²
   und A Alkyl mit 1, 2, 3 oder 4 C-Atomen bedeuten,
   umsetzt,
   und/oder dass man in einer Verbindung der Formel I gemäß Anspruch 1 einen oder mehrere Rest(e) R¹,R² und/oder R³ in einen oder mehrere Rest(e) R¹,R² und/oder R³ umwandelt,
   indem man beispielsweise
   i) eine Alkylsulfanylgruppe in ein Amin umwandelt,
   ii) einen Ester zur Säure hydrolysiert, zum Aldehyd oder Alkohol reduziert,
   iii) ein Nitril zum Aldehyd oder Amin reduziert,
      und/oder
      eine Base oder Säure der Formel I in eines ihrer Salze umwandelt.

Vor- und nachstehend haben die Reste R¹, R², R³, R⁴, B und X die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch Cycloalkyl.
Cycloalkyl bedeutet vorzugsweise Cyclopropyl, Cyclobutyl, Cylopentyl, Cyclohexyl oder Cycloheptyl.

Alkylen ist vorzugsweise unverzweigt und bedeutet bevorzugt Methylen, Ethylen, Propylen, Butylen oder Pentylen.

R¹ bedeutet vorzugsweise A, OH, NH₂, -(CH₂)ₘ-Ar', wie z.B. unsubstituiertes oder ein-, zwei- oder dreifach durch Hal, OA, A oder COOA substituiertes Phenyl, oder -(CH₂)ₘ-Het², wie z.B. Thienyl, Furyl, Imidazolyl, Pyrrolyl, Thiazolyl oder Pyridyl.
Wenn X C bedeutet, dann bedeutet R¹ vorzugsweise A, OH, -(CH₂)ₘ-Ar', wie z.B. unsubstituiertes oder ein-, zwei- oder dreifach durch Hal, OA, A oder COOA substituiertes Phenyl, oder -(CH₂)ₘ-Het², wie z.B. Thienyl, Furyl, Imidazolyl, Pyrrolyl, Thiazolyl oder Pyridyl.
Wenn X N bedeutet, dann bedeutet R¹ vorzugsweise NH₂.

Wenn X C bedeutet, dann bedeutet R² vorzugsweise H, CN, (CH₂)ₒAr", (CH₂)ₒCOOA oder SO₂A, wobei Ar" vorzugsweise unsubstituiertes oder ein-, zwei- oder dreifach durch Hal oder OA substituiertes Phenyl bedeutet; o bedeutet vorzugsweise 0 oder 1.

R³ bedeutet vorzugsweise H, A, -S-A, Phenyl, NH-Benzyl, -(CH₂)ₚ-Het, NH-(CH₂)ₚ-Het, NA₂, NH-Alkylen-NA₂ oder NA-Alkylen-NA₂, wobei Het vorzugsweise einen einkernigen gesättigten oder aromatischen Heterocyclus mit 1 bis 3 N- und/oder O-Atomen bedeutet, der unsubstituiert oder ein-, zwei- oder dreifach durch Hal, A, NHA, NA₂, COOA, Benzyl, - (CH₂)ₜ-OH oder -(CH₂)ₚ-Het¹ substituiert sein kann;
in diesem Zusammenhang bedeutet Het¹ vorzugsweise einen unsubstituierten einkernigen gesättigten oder aromatischen Heterocyclus mit 1 bis 2 N- und/oder O-Atomen, oder 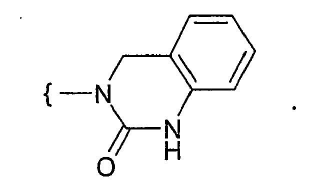 Het¹ bedeutet insbesondere Morpholinyl, Pyrrolidinyl, Piperidinyl, Piperazinyl, Pyrazinyl, Pyridyl, Furyl, Thienyl oder 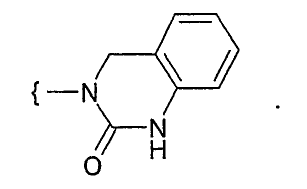 Het¹ bedeutet insbesondere Morpholinyl, Pyrrolidinyl, Pyridyl, oder 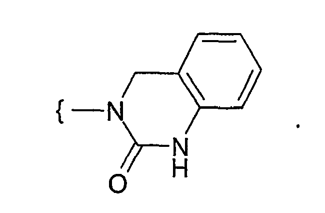 Ar bedeutet vorzugsweise unsubstituiertes oder ein-, zwei- oder dreifach durch Hal, A, OH, OA, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl.
Aryl bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p-Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5-oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Het bedeutet vorzugsweise einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, NHA, NA₂, OA, COOA, CN, -(CH₂)ₚ-Ar, -(CH₂)ₜ-OH, -(CH₂)ₚ-Het¹ oder Carbonylsauerstoff (=O) substituiert sein kann.
Het bedeutet besonders bevorzugt einen einkernigen gesättigten oder aromatischen Heterocyclus mit 1 bis 3 N- und/oder O-Atomen, der unsubstituiert oder ein-, zwei- oder dreifach durch Hal, A, NHA, NA₂, COOA, Benzyl, -(CH₂)ₜ-OH oder -(CH₂)ₚ-Het¹ substituiert sein kann; wobei Het¹ vorzugsweise einen unsubstituierten einkernigen gesättigten oder aromatischen Heterocyclus mit 1 bis 3 N- und/oder O-Atomen, oder 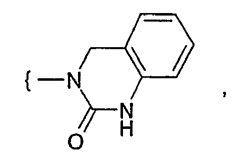 bedeutet.

Ungeachtet weiterer Substitutionen, bedeutet unsubstituiertes Het z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxad iazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder - 4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro- benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

In einer weiteren Ausführungsform bedeutet Het besonders bevorzugt Piperazinyl, Piperidinyl, Morpholinyl, Pyrrolidinyl, Pyridyl oder Furyl, die unsubstituiert sind oder ein-, zwei- oder dreifach durch Hal, A, NHA, NA₂, COOA, Benzyl, -(CH₂)ₜ-OH oder -(CH₂)ₚ-Het¹ substituiert sein können, wobei Het¹ vorzugsweise Morpholinyl, Pyrrolidinyl, Pyridyl oder 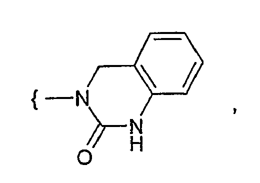 bedeutet.

Ungeachtet weiterer Substitutionen, bedeutet unsubstituiertes Het¹ z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het¹ kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl oder

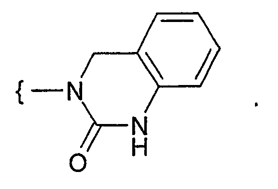

Ungeachtet weiterer Substitutionen durch A, bedeutet unsubstituiertes Het² z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Het² bedeutet vorzugsweise einen unsubstituierten einkernigen aromatischen Heterocyclus mit 1-2 N-, O- und/oder S-Atomen.

Ungeachtet weiterer Substitutionen durch A, bedeutet unsubstituiertes Het³ z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, - 4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het³ kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder-5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl oder auch 3,4-Dihydro-2H-1,5-benzodioxepin-6- oder -7-yl, ferner bevorzugt 2,3-Dihydrobenzofuranyl.

Het⁴ bedeutet vorzugsweise einen einkernigen gesättigten oder aromatischen Heterocyclus mit 1 bis 3 N-, O- und/oder S-Atomen, der unsubstituiert oder ein-, zwei- oder dreifach durch A, CONH₂, CONHA, CONA₂ oder Ar² substituiert sein kann, wobei Ar² vorzugsweise unsubstituiertes oder ein-, zwei- oder dreifach durch A substituiertes Phenyl bedeutet.

Het⁴ bedeutet besonders bevorzugt unsubstituiertes oder einfach durch CONHA, A und/oder Ar² substituiertes Pyridyl, Benzo[1,2,5]thiadiazol-5-yl, Piperazin, Thiazol oder Imidazol, wobei Ar² vorzugsweise unsubstituiertes oder ein-, zwei- oder dreifach durch A substituiertes Phenyl bedeutet.

In der Bedeutung für Ar¹ bedeutet Piperazin-diyl vorzugsweise Piperazin-1,4-diyl.
Hal bedeutet vorzugsweise F, Cl oder Br, aber auch I, besonders bevorzugt F oder Cl.

Für die gesamte Formel I gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I, worin X C bedeutet, können vorzugsweise erhalten werden, indem man Verbindungen der Formel II mit Verbindungen der Formel IIIa, IIIb oder IIIc umsetzt.

Die Verbindungen der Formel II sind neu, die der Formel IIIa, IIIb und IIIc sind in der Regel bekannt.

Die Umsetzung erfolgt in der Regel in einem inerten Lösungsmittel, gegebenenfalls in Gegenwart einer organischen Base wie Triethylamin, Dimethylanilin, Pyridin oder Chinolin. Die Reaktionszeit liegt je nach den angewendeten Bedingungen zwischen einigen Minuten und 14 Tagen, die Reaktionstemperatur zwischen etwa 0° und 180°, normalerweise zwischen 25° und 160°, besonders bevorzugt zwischen 60 und 160°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I, worin X N und R¹ NH₂ bedeuten, können weiter vorzugsweise erhalten werden, indem man Verbindungen der Formel II mit Verbindungen der Formel IIId umsetzt. Die Verbindungen der Formel IIId sind in der Regel bekannt.
Die Umsetzung erfolgt in der Regel in einem inerten Lösungsmittel und unter Bedingungen wie oben angegeben.

Verbindungen der Formel I, worin
- X: N,
- R¹: H, A, -(CH₂)ₘ-Ar oder -(CH₂)ₘ-Het²,
- R³: -S-A bedeuten, können weiter vorzugsweise erhalten werden,
indem man Verbindungen der Formel II mit Verbindungen der Formel IIIe umsetzt. Die Verbindungen der Formel IIId sind in der Regel bekannt. Die Umsetzung erfolgt in der Regel in einem inerten Lösungsmittel und unter Bedingungen wie oben angegeben.

Es ist ferner möglich, eine Verbindung der Formel I in eine andere Verbindung der Formel I umzuwandeln, indem man einen oder mehrere Rest(e) R¹, R² oder R³ in einen oder mehrere andere Reste R¹, R² oder R³ umwandelt, z.B. indem man
a) eine Alkylsulfanylgruppe in ein Amin umwandelt,
b) Nitrogruppen, beispielsweise durch Hydrierung an Raney-Nickel oder Pd-Kohle in einem inerten Lösungsmittel wie Methanol oder Ethanol, zu Aminogruppen reduziert,
b) eine Estergruppe in eine Carboxygruppe umwandelt,
c) eine Aminogruppe durch reduktive Aminierung in ein alkyliertes Amin umwandelt und/oder
d) Carboxygruppen durch Umsetzung mit Alkoholen verestert.

Die Umwandlung einer Alkylsulfanylgruppe in ein Amin erfolgt durch Umsetzung der Alkylsulfanylverbindung mit dem entsprechenden Amin in einem inerten Lösungsmittel. Die Reaktionszeit liegt je nach den angewendeten Bedingungen zwischen einigen Minuten und 14 Tagen, die Reaktionstemperatur zwischen etwa 0° und 180°, normalerweise zwischen 25° und 160°, besonders bevorzugt zwischen 60 und 160°C.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und /oder Hydroxygruppe durch Solvolyse oder Hydrogenolyse nach üblichen Methoden in Freiheit gesetzt werden. Dies kann z.B. mit NaOH oder KOH in Wasser, Wasser-THF oder Wasser-Dioxan bei Temperaturen zwischen 0 und 100° erfolgen.

Die Reduktion eines Esters zum Aldehyd oder zum Alkohol, oder die Reduktion eines Nitrils zum Aldehyd oder Amin erfolgt nach Methoden wie sie dem Fachmann bekannt sind und in Standardwerken der organischen Chemie beschrieben sind.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I gemäß Anspruch 1 werden größtenteils konventionell hergestellt. Sofern die Verbindung der Formel I gemäß Anspruch 1 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I gemäß Anspruch 1, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I gemäß Anspruch 1 mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I gemäß Anspruch 1 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservieru ingsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I gemäß Anspruch 1 sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I gemäß Anspruch 1 hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per* se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I gemäß Anspruch 1 und/oder ihre pharmazeutisch verwendbaren Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I gemäß Anspruch 1 und/oder ihrer pharmazeutisch verwendbaren Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I gemäß Anspruch 1 und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom. Ebenfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I gemäß Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I gemäß Anspruch 1 und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I gemäß Anspruch 1 können an Patienten zur Behandlung von Krebs verabreicht werden. Die vorliegenden Verbindungen hemmen die Tumorangiogenese und beeinflussen so das Wachstum von Tumoren (J. Rak et al. Cancer Research, 55:4575-4580, 1995). Die angiogenesehemmenden Eigenschaften der vorliegenden Verbindungen der Formel I eignen sich auch zur Behandlung bestimmter Formen von Blindheit, die mit Retina-Gefäßneubildung in Zusammenhang stehen.
Die Verbindungen der Formel I gemäß Anspruch 1 eignen sich auch zur Behandlung bestimmter Knochen-Pathologien wie Osteosarkom, Osteoarthritis und Rachitis, die auch unter der Bezeichnung onkogene Osteomalazie bekannt ist (Hasegawa et al., Skeletal Radiol. 28, S.41-45, 1999; Gerber et al., Nature Medicine, Bd. 5, Nr. 6, S.623-628, Juni 1999). Da der VEGF durch den in reifen Osteoklasten exprimierten KDR/Flk-1 direkt die osteoklastische Knochenresorption fördert (FEBS Let. 473:161-164 (2000); Endocrinology, 141:1667 (2000)), eignen sich die vorliegenden Verbindungen auch zur Behandlung und Vorbeugung von Leiden, die mit Knochenresorption in Zusammenhang stehen, wie Osteoporose und Morbus Paget.
Die Verbindungen können dadurch, dass sie zerebrale Ödeme, Gewebeschädigung und ischämiebedingte Reperfusionsverletzungen reduzieren, auch zur Verringerung oder Vorbeugung von Gewebeschäden, die nach zerebralen ischämischen Ereignissen wie Gehirnschlag auftreten, verwendet werden (Drug News Perspect 11:265-270 (1998); J. Clin. Invest. 104:1613-1620 (1999)).

Wie erläutert, sind die Signalwege für verschiedene Erkrankungen relevant. Folglich eignen sich die erfindungsgemäßen Verbindungen, indem sie mit einem oder mehreren dieser Signalwege interagieren, zur Vorbeugung und/oder Behandlung von Erkrankungen, die von diesen Signalwegen abhängig sind.

Die erfindungsgemäßen Verbindungen sind bevorzugt Kinase-Modulatoren und bevorzugter Kinase-Inhibitoren. Erfindungsgemäß umfassen Kinasen, sind aber nicht beschränkt auf, eine oder mehrere Tie-Kinasen, eine oder mehrere VEGFR-Kinasen, eine oder mehrere PDGFR-Kinasen, p38-Kinase und/oder SAPK2alpha.

Gegenstand der Erfindung ist somit die Verwendung von Verbindungen der Formel I, gemäß Anspruch 1 sowie ihrer pharmazeutisch verwendbaren Solvate und Stereoisomere, einschließlich deren Mischungen in allen Verhältnissen, zur Herstellung eines Arzneimittels zur Behandlung von Krankheiten, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung ist weiterhin die Verwendung der Verbindungen der Formel I zur Behandlung einer Krankheit, an der Angiogenese beteiligt ist.

Vorzugsweise handelt es sich bei der Krankheit um eine Augenkrankheit.

Gegenstand der Erfindung ist weiterhin die Verwendung zur Behandlung von Retina-Vaskularisierung, diabetischer Retinopathie, altersbedingter Makula-Degeneration und/oder Entzündungskrankheiten.

Die Entzündungskrankheit ist vorzugsweise ausgewählt aus der Gruppe rheumatoide Arthritis, Schuppenflechte, Kontaktdermatitis und Spät-Typ der Überempfindlichkeitsreaktion stammt.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis stammt.

Die Verbindungen der Formel I gemäß Anspruch 1 können auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinblocker (wie sie weiter unten definiert werden), wie αvβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Hemmer und ATP-Protonenpumpenhemmer enthalten.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den
Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Gegenstand der Erfindung ist ferner die Verwendung der Verbindungen der Formel I gemäß Anspruch 1 zur Herstellung eines Arzneimittels zur Behandlung von Krankheiten, wobei die Krankheit durch gestörte Angiogenese gekennzeichnet ist. Bei der Krankheit handelt es sich vorzugsweise um Krebserkrankungen.
Die gestörte Angiogenese resultiert vorzugsweise aus einer gestörten VEGFR-1- , VEGFR-2- und/oder VEGFR-3-Aktivität.

Besonders bevorzugt ist daher auch die Verwendung der erfindungsgemäßen Verbindungen zur Herstellung eines Arzneimittels zur Inhibierung der VEGFR-2-Aktivität.

### ASSAYS

Die in den Beispielen beschriebenen Verbindungen der Formel I gemäß Anspruch 1 wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Allgemein sind erfindungsgemäße Verbindungen als geeignete Kinase-Modulatoren und besonders als geeignete erfindungsgemäße Kinase-Inhibitoren anzusehen, wenn sie eine Wirkung oder eine Aktivität auf eine oder mehrere Kinasen, bevorzugt auf eine oder mehrere Raf-Kinasen, zeigen, die bevorzugt, bestimmt als IC₅₀-Wert, im Bereich von 100 µmol oder darunter, bevorzugt 10 µmol oder darunter, bevorzugter im Bereich von 3 µmol oder darunter, noch bevorzugter im Bereich von 1 µmol oder darunter und am stärksten bevorzugt im nanomolaren Bereich liegt. Besonders bevorzugt für die erfindungsgemäße Verwendung sind Kinase-Inhibitoren, wie vorstehend/nachstehend definiert, die eine Aktivität, bestimmt als IC₅₀-Wert, auf eine oder mehrere Raf-Kinasen, im Bereich von 0,5 µmol oder darunter und besonderes im Bereich von 0,1 µmol oder darunter zeigen. In vielen Fällen ist ein IC₅₀-Wert am unteren Ende der angegebenen Bereiche vorteilhaft, und in einigen Fällen ist es sehr wünschenswert, dass der IC₅₀-Wert so klein wie möglich ist oder dass die IC₅₀-Werte so klein wie möglich sind, aber gewöhnlich sind IC₅₀-Werte, die zwischen den vorstehend angegebenen oberen Grenzen und einer unteren Grenze im Bereich von 0,0001 µmol, 0,001 µmol, 0,01 µmol oder sogar über 0,1 µmol liegen, ausreichend, damit sie die gewünschte pharmazeutische Aktivität zeigen. Die gemessenen Aktivitäten können jedoch je nach dem entsprechenden gewählten Testsystem oder Assay schwanken.

Alternativ kann die vorteilhafte biologische Aktivität der erfindungsgemäßen Verbindung in *in vitro* Assays, wie *in vitro* Proliferationsassays oder *in vitro* Wachstumsassays, demonstriert werden. Geeignete *in vitro* Assays sind im Stand der Technik bekannt, zum Beispiel aus der hier zitierten Literatur und den in der Literatur zitierten Bezugsstellen, oder können wie nachstehend beschrieben durchgeführt oder auf dazu analoge Weise entwickelt und/oder durchgeführt werden.

Als Beispiel für einen *in vitro* Wachstumsassay können menschliche Tumorzelllinien, zum Beispiel HCT116, DLD-1 oder MiaPaCa, die mutierte K-ras-Gene enthalten, in Standard-Proliferationsassays, zum Beispiel für das Verankerungs-anhängige Wachstum auf Kunststoff oder das Verankerungs-unabhängige Wachstum in Weichagar, verwendet werden. Menschliche Tumorzelllinien sind kommerziell erhältlich, zum Beispiel von ATCC (Rockville, MD), und können gemäß im Stand der Technik bekannten Verfahren, zum Beispiel in RPMI mit 10% hitzeinaktiviertem fötalem Rinderserum und 200 mM Glutamin, kultiviert werden. Zellkulturmedien, fötales Rinderserum und Hilfsstoffe sind kommerziell erhältlich, zum Beispiel von Invitrogen/Gibco/BRL (Karlsruhe, BRD) und/oder QRH Biosciences (Lenexa, KS). In einem Standard-Proliferationsassay für Verankerungsanhängiges Wachstum kann man 3 x 10³ Zellen in Gewebekulturplatten mit 96 Vertiefungen überimpfen und sich anheften lassen, zum Beispiel über Nacht bei 37 °C in einem Brutschrank bei 5 % CO₂. Die Verbindungen können in Medien in Verdünnungsreihen titriert und zu den Zellkulturen in 96 Vertiefungen hinzugefügt werden. Man lässt die Zellen wachsen, zum Beispiel 1 bis 5 Tage, gewöhnlich mit einem Nachfüllen von frischen, die Verbindung enthaltenden Medien bei etwa der Hälfte der Dauer des Wachstumszeitraums, zum Beispiel an Tag 3, wenn man die Zellen für 5 Tage wachsen lässt. Die Proliferation kann durch im Stand der Technik bekannte Verfahren überwacht werden, wie durch Messung der Stoffwechselaktivität, zum Beispiel mit einem colorimetrischen Standard-XXT-Assay (Boehringer, Mannheim), der durch ein Standard-ELISA-Plattenlesegerät bei OD 490/560 gemessen wird, durch Messen des ³H-Thymidin-Einbaus in DNA nach einer 8stündigen Kultur mit 1 µCi ³H-Thymidin, Ernten der Zellen auf Glasfasermatten unter Verwendung einer Zellerntevorrichtung und Messen des ³H-Thymidin-Einbau durch Flüssigszintillationszählung oder durch Färbetechniken, wie Kristallviolettfärbung. Andere geeignete zelluläre Assaysysteme sind im Stand der Technik bekannt.

Alternativ können für Verankerungs-unabhängiges Zellwachstum 1 x 10³ bis 3 x 10³ Zellen in 0,4 % Seaplaque-Agarose in RPMI-Vollmedien ausplattiert werden, wobei eine Bodenschicht, die nur 0,64 % Agar in RPMI-Vollmedien enthält, zum Beispiel in Gewebekulturschalen mit 24 Vertiefungen, überschichtet wird. Vollmedien plus Verdünnungsreihen von Verbindungen können zu den Vertiefungen gegeben und, zum Beispiel bei 37 °C in einem Brutschrank bei 5 % CO₂, für einen ausreichenden Zeitraum inkubiert werden, zum Beispiel 10-14 Tage, bevorzugt unter wiederholtem Nachfüllen von frischen, die Verbindung enthaltenden Medien, üblicherweise in Abständen von 3-4 Tagen. Koloniebildung und Gesamtzellmasse können überwacht werden, die durchschnittliche Koloniegröße und die Anzahl der Kolonien können gemäß im Stand der Technik bekannten Verfahren quantifiziert werden, zum Beispiel unter Verwendung von "Image Capture"-Technologie und Bildanalyse-Software. "Image Capture"-Technologie und Bildanalyse-Software, wie Image Pro Plus oder media Cybernetics.

### VEGF-Rezeptorkinase-Assay

Die VEGF-Rezeptorkinaseaktivität wird durch Einbau von radioaktiv markiertem Phosphat in 4:1 Polyglutaminsäure/Tyrosin-Substrat (pEY) bestimmt. Das phosphorylierte pEY-Produkt wird auf einer Filtermembran festgehalten, und der Einbau des radioaktiv markierten Phosphats wird durch Szintillationszählung quantitativ bestimmt.

### MATERIALIEN

### VEGF-Rezeptorkinase

Die intrazelluläre-Tyrosinkinase-Domänen des menschlichen KDR (Terman, B. I. et al. Oncogene (1991) Bd. 6, S. 1677-1683.) und Flt-1 (Shibuya, M. et al. Oncogene (1990) Bd. 5, S. 519-524) wurden als Glutathion-S-transferase (GST)-Genfusionsproteine kloniert. Dies geschah durch Klonieren der Zytoplasma-Domäne der KDR-Kinase als leserastergerechte Fusion am Carboxy-Terminus des GST-Gens. Die löslichen rekombinanten GST-Kinasedomäne-Fusionsproteine wurden in *Spodoptera frugiperda* (Sf21) Insektenzellen (Invitrogen) unter Verwendung eines Baculovirus-Expressionsvektors (pAcG2T, Pharmingen) exprimiert.

### Lysepuffer

50 mM Tris pH 7,4, 0,5 M NaCl, 5 mM DTT, 1 mM EDTA, 0,5% Triton X-100, 10% Glycerin, je 10 mg/ml Leupeptin, Pepstatin und Aprotinin sowie 1 mM Phenylmethylsulfonylfluorid (alle von Sigma).
Waschpuffer 50 mM Tris pH 7,4, 0,5 M NaCl, 5 mM DTT, 1 mM EDTA, 0.05% Triton X-100, 10% Glycerin, je 10 mg/ml Leupeptin, Pepstatin und Aprotinin sowie 1 mM Phenylmethylsulfonylfluorid.
Dialysepuffer
50 mM Tris pH 7,4, 0,5 M NaCl, 5 mM DTT, 1 mM EDTA, 0.05% Triton X-100, 50% Glycerin, je 10 mg/ml Leupeptin, Pepstatin und Aprotinin sowie 1 mM Phenylmethylsulfonylfluorid.
10x Reaktionspuffer
200 mM Tris, pH 7,4, 1,0 M NaCl, 50 mM MnCl₂, 10 mM DTT und 5 mg/ml Rinderserumalbumin [bovine serum albumin = BSA] (Sigma). Enzymverdünnungspuffer
50 mM Tris, pH 7,4, 0,1 M NaCl, 1 mM DTT, 10% Glycerin, 100 mg/ml BSA.
10× Substrat
750 µg/ml Poly(glutaminsäure/Tyrosin; 4:1) (Sigma).
Stopp-Lösung
30% Trichloressigsäure, 0,2 M Natriumpyrophosphat (beide von Fisher). Waschlösung
15% Trichloressigsäure, 0,2 M Natriumpyrophosphat.
Filterplatten
Millipore #MAFC NOB, GF/C 96-Well-Glasfaserplatte.
Verfahren A - Proteinaufreinigung
1. Die Sf21-Zellen wurden mit dem rekombinanten Virus bei einer m.o.i. (Multiplizität der Infektion) von 5 Viruspartikeln/Zelle infiziert und 48 Stunden lang bei 27°C gezüchtet.
2. Alle Schritte wurden bei 4°C durchgeführt. Die infizierten Zellen wurden durch Zentrifugieren bei 1000xg geerntet und 30 Minuten bei 4°C mit 1/10 Volumen Lysepuffer lysiert und anschließend 1 Stunde lang bei 100.000×g - zentrifugiert. Der Überstand wurde dann über eine mit Lysepuffer äquilibrierte Glutathion-Sepharose-Säure (Pharmacia) gegeben und mit 5 Volumina des gleichen Puffers und anschließend 5 Volumina Waschpuffer gewaschen. Das rekombinante GST-KDR-Protein wurde mit Waschpuffer/10 mM reduziertem Glutathion (Sigma) eluiert und gegen Dialysepuffer dialysiert.
Verfahren B - VEGF-Rezeptorkinase-Assay
1. Assay mit 5 µl Hemmstoff oder Kontrolle in 50% DMSO versetzen.
2. Mit 35 µl Reaktionsmischung, die 5 µl 10× Reaktionspuffer, 5 µl 25 mM ATP/10 µCi[³³ P]ATP (Amersham) und 5 µl 10× Substrat enthält, versetzen.
3. Reaktion durch Zugabe von 10 µl KDR (25 nM) in Enzymverdünnungspuffer starten.
4. Mischen und 15 Minuten lang bei Raumtemperatur inkubieren.
5. Reaktion durch Zugabe von 50 µl Stopp-Lösung stoppen.
6. 15 Minuten lang bei 4°C inkubieren.
7. 90 µl-Aliquot auf Filterplatte überführen.
8. Absaugen und 3 Mal mit Waschlösung waschen.
9. 30 µl Szintillations-Cocktail zugeben, Platte verschließen und in einem Szintillations-Zähler Typ Wallac Microbeta zählen.
Mitogenese-Assay an menschlichen Nabelschnurvenenendothelzellen
Die Expression von VEGF-Rezeptoren, die mitogene Reaktionen auf den Wachstumsfaktor vermitteln, ist größtenteils auf Gefäßendothelzellen beschränkt. Kultivierte menschliche Nabelschnurvenenendothelzellen (HUVECs) proliferieren als Reaktion auf Behandlung mit VEGF und können als Assaysystem zur quantitativen Bestimmung der Auswirkungen von KDR-Kinasehemmern auf die Stimulation des VEGF verwendet werden. In dem beschriebenen Assay werden Einzelzellschichten von HUVECs im Ruhezustand 2 Stunden vor der Zugabe von VEGF oder "basic fibroblast growth factor" (bFGF) mit dem Konstituens oder der Testverbindung behandelt. Die mitogene Reaktion auf VEGF oder bFGF wird durch Messung des Einbaus von [³H]Thymidin in die Zell-DNA bestimmt.

### Materialien

### HUVECs

Als Primärkulturisolate tiefgefrorene HUVECs werden von Clonetics Corp bezogen. Die Zellen werden im Endothel-Wachstumsmedium (Endothelial Growth Medium = EGM; Clonetics) erhalten und in der 3. - 7. Passage für die Mitogenitätsassays verwendet.

### Kulturplatten

NUNCLON 96-Well-Polystyrol-Gewebekulturplattten (NUNC #167008).

### Assay-Medium

Nach Dulbecco modifiziertes Eagle-Medium mit 1 g/ml Glucose (DMEM mit niedrigem Glucosegehalt; Mediatech) plus 10% (v/v) fötales Rinderserum (Clonetics).

### Testverbindungen

Mit den Arbeitsstammlösungen der Testverbindungen wird mit 100% Dimethylsulfoxid (DMSO) solange eine Reihenverdünnung durchgeführt, bis ihre Konzentrationen um das 400-fache höher als die gewünschte Endkonzentration sind. Die letzten Verdünnungen (Konzentration 1×) werden unmittelbar vor Zugabe zu den Zellen mit Assay-Medium hergestellt.
10× Wachstumsfaktoren
Lösungen des menschlichen VEGF 165 (500 ng/ml; R&D Systems) und bFGF (10 ng/ml; R&D Systems) werden mit Assay-Medium hergestellt. 10× [³H]-Thymidin
[Methyl-³H]-Thymidin (20 Ci/mmol; Dupont-NEN) wird mit DMEM-Medium mit niedrigem Glucosegehalt auf 80µCi/ml verdünnt.

### Zellwaschmedium

Hank's balanced salt solution (Mediatech) mit 1 mg/ml Rinderserumalbumin (Boehringer-Mannheim).
Zell-Lyse-Lösung
1 N NaOH, 2% (w/v) Na₂CO₃.

### Verfahren 1

In EGM gehaltene HUVEC-Einzelzellschichten werden durch Trypsinbehandlung geerntet und in einer Dichte von 4000 Zellen pro 100 µl Assay-Medium pro Näpfchen in 96-Well-Platten überimpft. Das Wachstum der Zellen wird 24 Stunden bei 37°C in einer 5% CO₂ enthaltenden feuchten Atmosphäre gestoppt.

### Verfahren 2

Das Wachstumsstoppmedium wird durch 100 µl Assay-Medium ersetzt, das entweder das Konstituens (0,25% [v/v] DMSO) oder die erwünschte Endkonzentration der Testverbindung enthält. Alle Bestimmungen werden in dreifacher Wiederholung durchgeführt. Die Zellen werden dann 2 Stunden bei 37°C/5% CO₂ inkubiert, so dass die Testverbindungen in die Zellen eindringen können.

### Verfahren 3

Nach 2-stündiger Vorbehandlung werden die Zellen durch Zugabe von 10 µl Assay-Medium, 10× VEGF-Lösung oder 10x bFGF-Lösung pro Näpfchen stimuliert. Die Zellen werden dann bei 37°C/5% CO₂ inkubiert.

### Verfahren 4

Nach 24 Stunden in Anwesenheit der Wachstumsfaktoren wird mit 10× [³H]-Thymidin (10 µl/well) versetzt.

### Verfahren 5

Drei Tage nach dem Versetzen mit [³H]-Thymidin wird das Medium abgesaugt und die Zellen werden zweimal mit Zellwaschmedium gewaschen (400 µl/well, anschließend 200 µl/well). Die gewaschenen, adhärenten Zellen werden dann durch Zugabe von Zell-Lyse-Lösung (100 µl/well) und 30-minutiges Erwärmen auf 37°C solubilisiert. Die Zell-Lysate werden in 7-ml-Szintillationsrährchen aus Glas, die 150 µl Wasser enthalten, überführt. Man versetzt mit dem Szintillations-Cocktail (5 ml/Röhrchen), und die mit den Zellen assoziierte Radioaktivität wird flüssigkeitsszintillationsspektroskopisch bestimmt.
Gemäß diesen Assays stellen die Verbindungen der Formel I VEGF-Hemmer dar und eignen sich daher zur Hemmung der Angiogenese, wie bei der Behandlung von Augenkrankheiten, z.B. diabetischer Retinopathie, und zur Behandlung von Karzinomen, z.B. festen Tumoren. Die vorliegenden Verbindungen hemmen die VEGF-stimulierte Mitogenese von kultivierten menschlichen Gefäßendothelzellen mit HK50-Werten von 0,01-5,0 µM. Diese Verbindungen sind im Vergleich zu verwandten Tyrosinkinasen (z.B. FGFR1 sowie Src-Familie; zur Beziehung zwischen Src-Kinasen und VEGFR-Kinasen siehe Eliceiri et al., Molecular Cell, Bd. 4, S.915-924, Dezember 1999) auch selektiv.

Die ***TIE-2***-Tests können z.B. analog der in WO 02/44156 angegebenen Methoden durchgeführt werden.
Der Assay bestimmt die inhibierende Aktivität der zu testenden Substanzen bei der Phosphorylierung des Substrats Poly(Glu, Tyr) durch Tie-2-Kinase in Gegenwart von radioaktivem ³³P-ATP. Das phosphorylierte Substrat bindet während der Inkubationszeit an die Oberfläche einer "flashplate"-Mikrotiterplatte. Nach Entfernen der Reaktionsmischung wird mehrmals gewaschen und anschließend die Radioaktivität an der Oberfläche der Mikrotiterplatte gemessen. Ein inhibierender Effekt der zu messenden Substanzen hat eine geringere Radioaktivität, verglichen mit einer ungestörten enzymatischen Reaktion, zur Folge.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:
Ethylacetat/Methanol 9:1.
Massenspektrometrie (MS): EI (Elektronenstoß-Ionisation) M⁺
FAB (Fast Atom Bombardment) (M+H)⁺
ESI (Electrospray lonization) (M+H)⁺
APCI-MS (atmospheric pressure chemical ionization - mass spectrometry) (M+H)⁺.

Bedingungen zur Bestimmung von R_{f}-Werten durch HPLC:
Anlage: HP Series 1100 mit Agilent 1100 Diode Array Detector (220 nm);
Säule: Chromolith Speed Rod RP18e, 50-4,6 mm;
Fluß: 2,4 mL/min;
Lösungsmittelverhältnis am Start:
Lösungsmittel (LM) A (Wasser + 0,01 % TFA): 80 %
Lösungsmittel B (Acetonitril + 0,01 % TFA): 20 %

### Zeittabelle

| Zeit [min] | LM A | LM B |
|---|---|---|
| 0 | 80 | 20 |
| 2,8 | 0 | 100 |
| 3,3 | 0 | 100 |
| 3,4 | 80 | 20 |
| 3,6 | 80 | 20 |

### Beispiele zur Herstellung von Ausgangsverbindungen der Formel 1-1

### Beispiel 1

### Herstellung von N-[4-(Pyridin-4-yloxy)-phenyl]-4H-[1,2,4]triazol-3,5-diamin ("1")

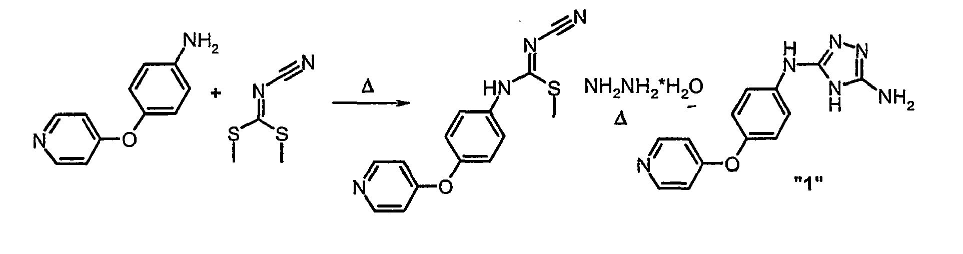

4-(Pyridin-4-yloxy)-phenylamin (9.80 g, 52.6 mmol) wird in Ethanol (250 mL) gelöst, Dimethyl-[N-cyandithioiminocarbonat] (7.75 g, 52.6mmol) wird zugefügt und 2 Tage unter Rückfluss gerührt. Die flüchtigen Bestandteile werden unter Vakuum vollständig entfernt, der Rückstand erneut in Ethanol (250 mL) aufgenommen, Hydrazinhydrat (50 mL) zugefügt und 2 h unter Rückfluss erhitzt. Das nach Abkühlung ausgefallene Produkt wird abfiltriert, mit Diethylether gewaschen und getrocknet. Man erhält 10.6 g (3.95 mmol, 75 %) "1 ", [M+H]⁺ 269, R_{f} 0,573 min.

Analog erhält man die nachstehenden Verbindungen
*N*-{4-[2-(N-Methylamino-carbonyl)-pyridin-4-yloxy]-phenyl}-4*H-*[1,2,4]triazol-3,5-diamin, [M+H]⁺ 326, R_{f} 0,884 min;
*N*-{3-[2-(N-Methylamino-carbonyl)-pyridin-4-yloxy]-phenyl}-4*H-*[1,2,4]triazol-3,5-diamin, R_{f} 1,058 min;
*N*-[4-(Pyridin-4-yloxy)-phenylmethyl]-4*H*-[1,2,4]triazol-3,5-diamin, [M+H]⁺ 283, R_{f} 0,510 min;
*N*-(5-Methyl-2-phenyl-2*H*-[1,2,3]triazol-4-ylmethyl)-4*H*-[1,2,4]triazol-3,5-diamin, [M+H]⁺ 271, R_{f} 1,169 min;
*N*-(2-Phenyl-thiazol-4-ylmethyl)-4*H*-[1,2,4]triazol-3,5-diamin, [M+H]⁺ 273, R_{f} 1,168 min;
*N*-[4-(2-Diethylamino-ethoxy)-phenyl]-4*H*-[1,2,4]triazol-3,5-diamin, R_{f} 0,462 min;
*N*-[4-(Benzo[1,2,5]thiadiazol-5-yloxy)-phenyl]-4*H*-[1,2,4]triazol-3,5-diamin, [M+H]⁺ 326, R_{f} 1,310 min;
*N*-[4-(Pyridin-4-ylsulfanyl)-phenyl]-4H-[1,2,4]triazol-3,5-diamin.

### Beispiel 2

### Herstellung von 5-Amino-3-[4-(pyridin-4-yloxy)-phenylamino]-1H-pyrazol-4-carbonitril ("2")

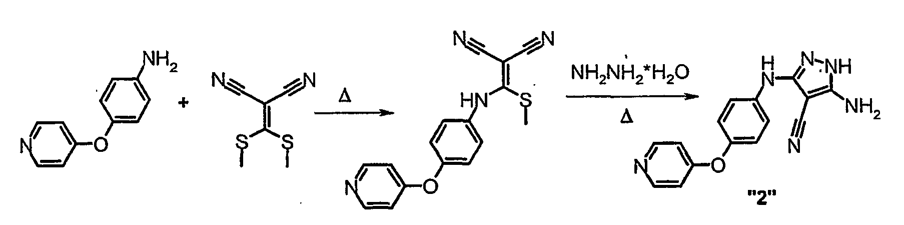

Analog zu Beispiel 1 wird 4-(Pyridin-4-yloxy)-phenylamin (10.2 g, 54.7 mmol) zuerst mit 3,3-Bis(methylthio)-2-cyanarylnitril (9.33 g, 54.7 mmol) und dann mit Hydrazinhydrat (50 mL) umgesetzt. Das nach Abkühlung ausgefallene Produkt wird abfiltriert, mit Diethylether gewaschen und getrocknet. Man erhält 13.6 g (46.5 mmol, 85 %) einer leicht braunen Substanz ("2"), R_{f} 0,607 min;

### Beispiel 3

### Herstellung von N*3*-[4-(Pyridin-4-yloxy)-phenyl]-1H-pyrazol-3,5-diamin ("3")

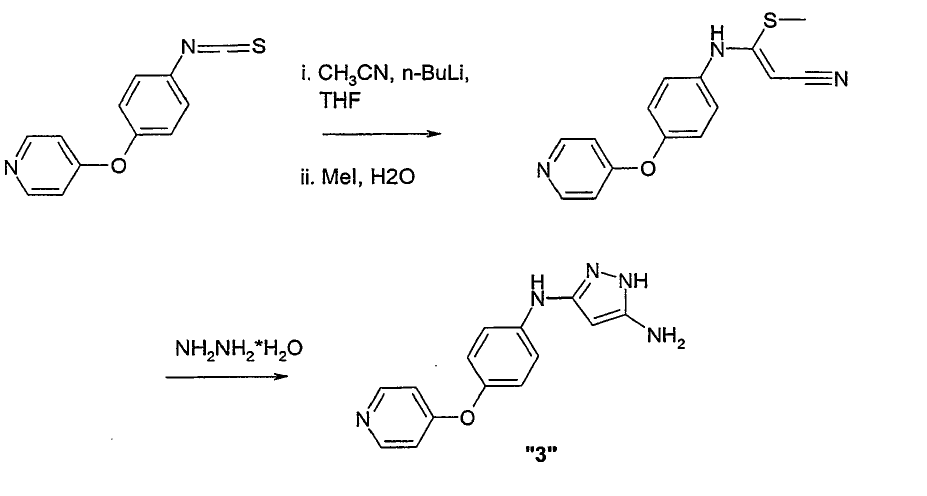

Acetonitril (0.79 mL, 15.0 mmol) wird in THF (tr., 20 mL) auf -78°C abgekühlt, n-BuLi-Lösung in Hexan (2.36 M, 5.10 mL, 12.0 mmol) langsam zugetropft und 30 min bei gleicher Temperatur gerührt. Das Isothiocyanat (0.50 g, 2.19 mmol, gelöst in THF, 8 mL) wird bei -78°C zugefügt, wobei sich ein Feststoff ausbildet, der sich auch nicht bei Erwärmung auf 0°C auflöst. Ethylacetat wird zugefügt und die organische Phase 3x mit Wasser gewaschen. Die wässrige Phase wird mit CH₃l (0.29 mL, 2.5 mmol) versetzt und 5 h bei RT gerührt. Die Lösung wird 2x mit Ethylacetat extrahiert, getrocknet und das Lösungsmittel entfernt. "3" wird als farbloser Feststoff erhalten (0.34 g, 1.2 mol, 55 %), [M+H]⁺ 268, R_{f} 0,544 min.

### Beispiele zur Herstellung von Verbindungen der Formel I gemäß Anspruch 1

### Beispiel 4 (Vergleichsbeispiel)

### Herstellung von (7-Phenyl-5-trifluormethyl-[1,2,4]triazolo[1,5-a]pyrimidin-2-yl)-[4-(pyridin-4-yloxy)-phenyl]-amin ("4")

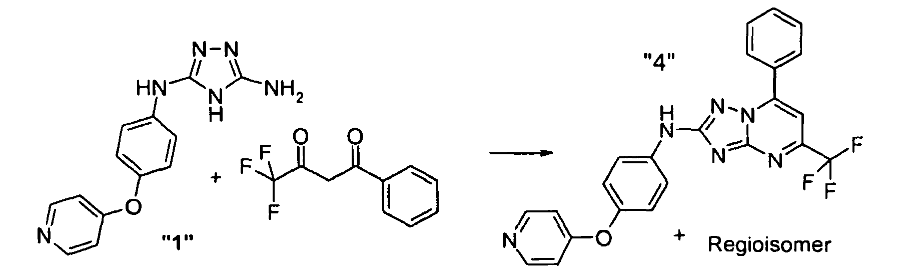

"1" (100 mg, 0.37 mol) und 4,4,4-Trifluor-1-phenyl-1,3-butandion (81 mg, 0.37 mmol) werden in Essigsäure (2 mL) 18 h in einem geschlossenen Gefäß auf 100°C erhitzt. Zu der abgekühlten Lösung wird Ethylacetat und Petrolether gegeben und der sich bildende Niederschlag abfiltriert, mit Diethylether gewaschen und getrocknet.
Man erhält 121 mg "4" * 0,6 Acetat (0.25 mmol, 68 %) als farblosen Feststoff; (M+H)⁺ 449.

Analog erhält man die nachstehenden Verbindungen

| Nr. | Struktur | (M+H)⁺ | |
|---|---|---|---|
| 28a | 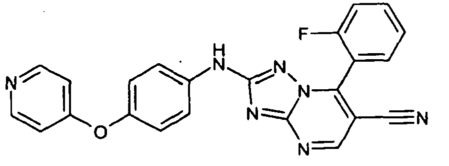 | 424 | |
| 28b | 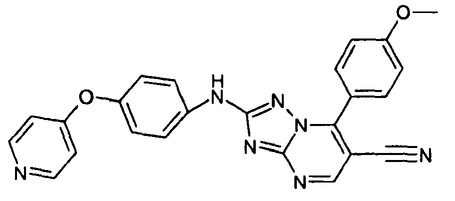 | 436 | |
| 28c | 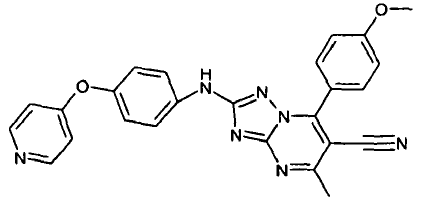 | 450 | |

### Beispiel 5

### Herstellung von 7-(4-Chlor-phenyl)-5-methylsulfanyl-2-[4-(pyridin-4-yloxy)-phenylamino]-[1,2,4]triazolo[1,5-a]pyrimidin-6-carbonitril ("76")

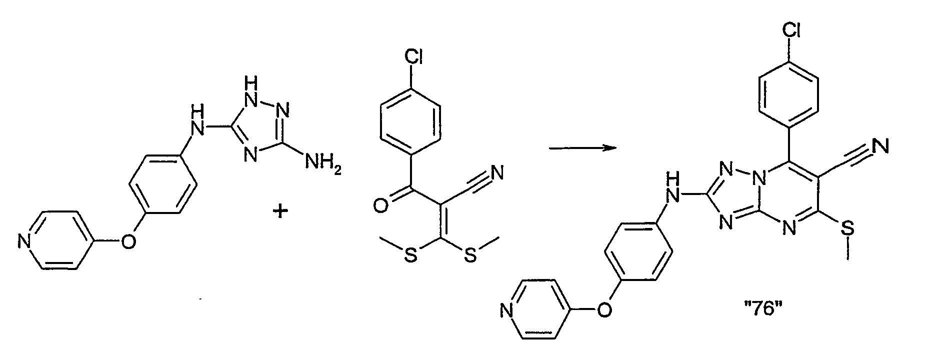

"1" (100 mg, 0.37 mol) und 2-(4-Chlor-benzoyl)-3,3-bis-methyl-sulfanyl-acrylonitril (114 mg, 0.40 mmol) werden in Ethanol (1 mL) 18 h in einem geschlossenen Gefäß auf 100°C erhitzt. Zu der abgekühlten Lösung wird Ethylacetat (5 mL) gegeben und der sich bildende Niederschlag abfiltriert, mit Diethylether gewaschen und getrocknet.
Man erhält 125 mg (0.26 mmol, 69 %) "76", [M+H]⁺ 486, als leicht bräunlichen Feststoff.

Analog erhält man die nachstehenden Verbindungen "77-125a"

| Nr. | Struktur | (M+H)⁺ | |
|---|---|---|---|
| 77 | 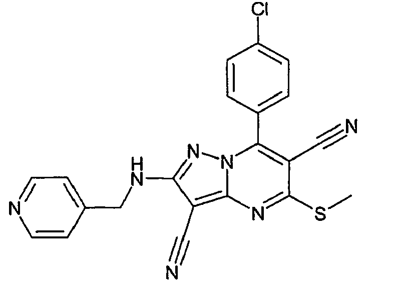 | 433 | |
| 78 | 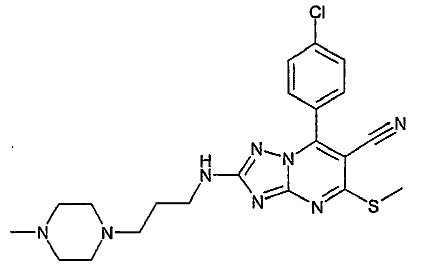 | 458 | |
| 79 | 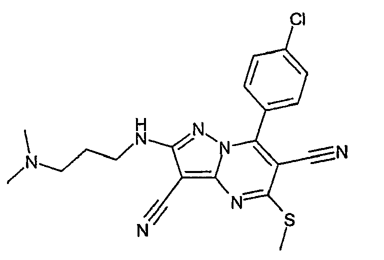 | 427 | |
| 80 | 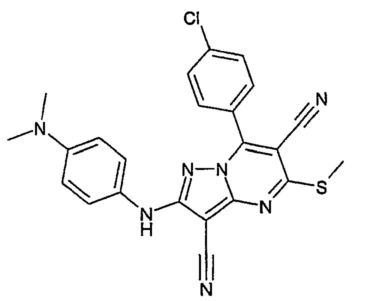 | 461 | |
| 81 | 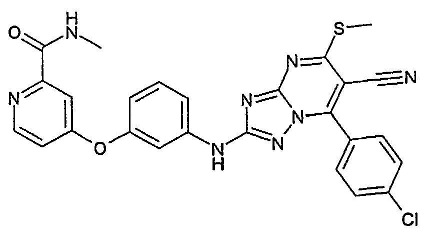 | 544 | |
| 82 | 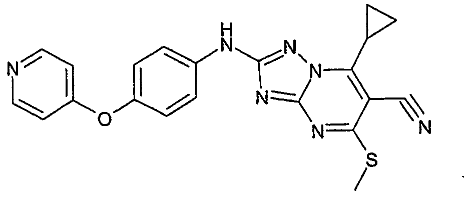 | 416 | |
| 83 | 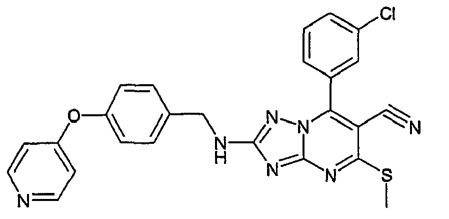 | 501 | |
| 84 | 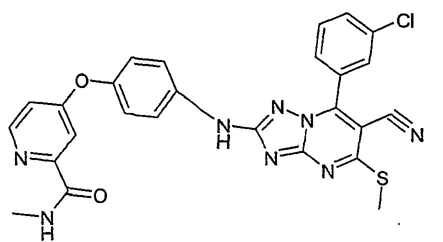 | 544 | |
| 85 | 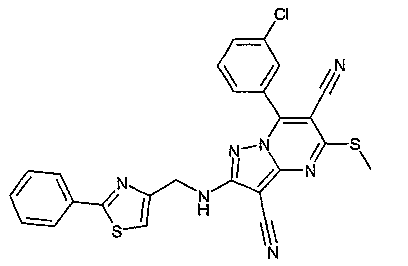 | 515 | |
| 86 | 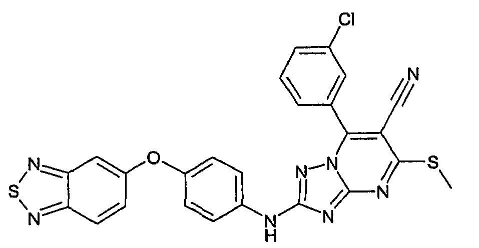 | 544 | |
| 87 | 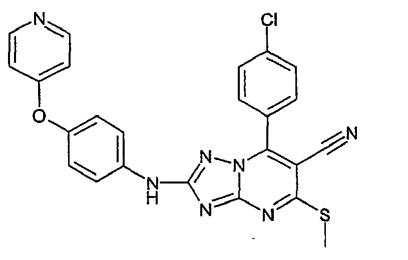 | 487 | |
| 88 | 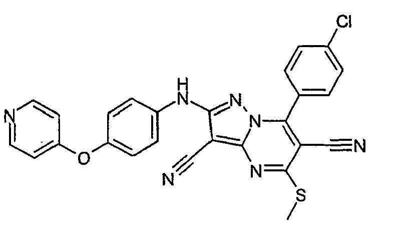 | 511 | |
| 89 | 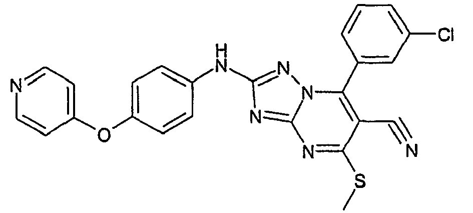 | 487 | |
| 90 | 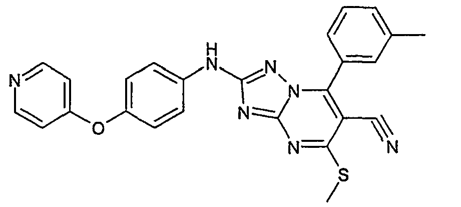 | 467 | |
| 91 | 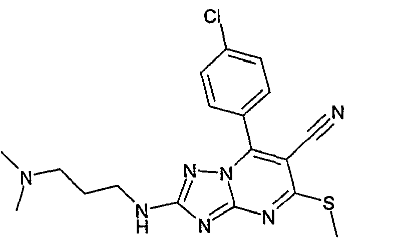 | 403 | |
| 92 | 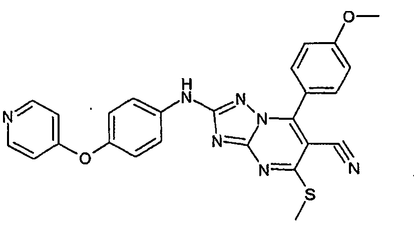 | 483 | |
| 93 | 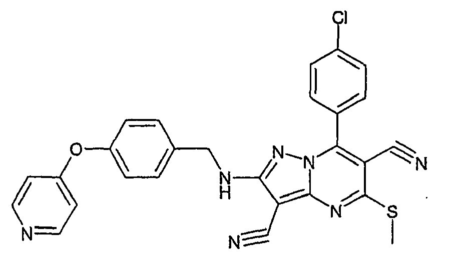 | 525 | |
| 94 | 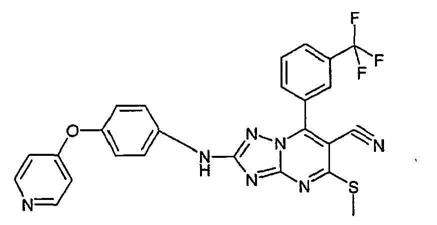 | 521 | |
| 95 | 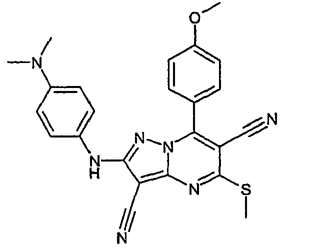 | 457 | |
| 96 | 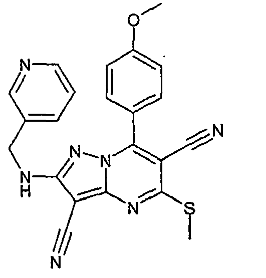 | 428 | |
| 97 | 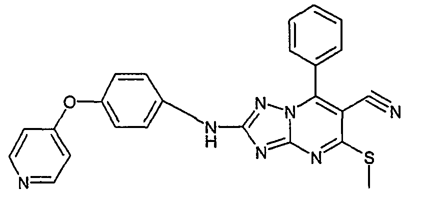 | 453 | |
| 98 | 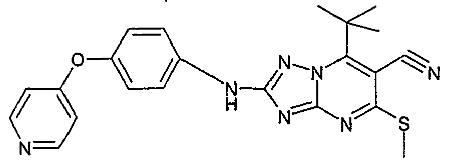 | 433 | |
| 99 | 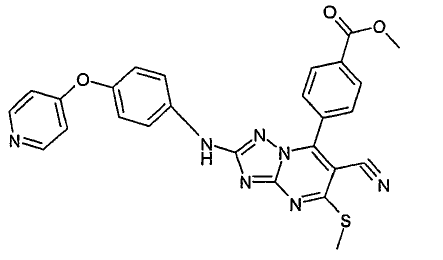 | 511 | |
| 100 | 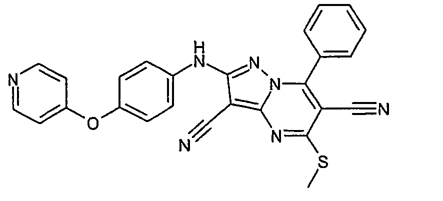 | 477 | |
| 101 | 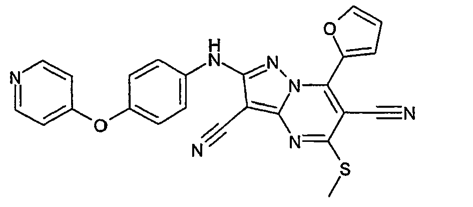 | 466 | |
| 102 | 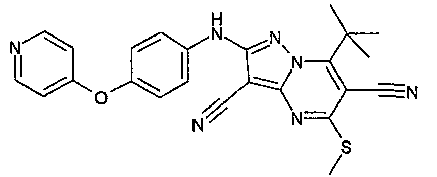 | 457 | |
| 103 | 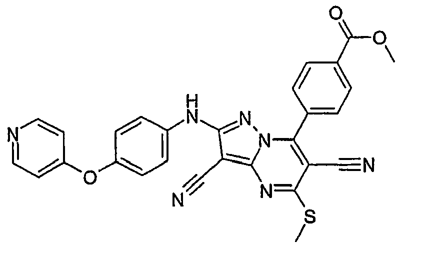 | 535 | |
| 104 | 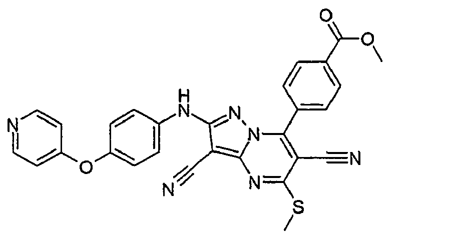 | 461 | |
| 105 | 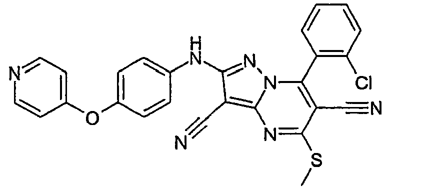 | 511 | |
| 106 | 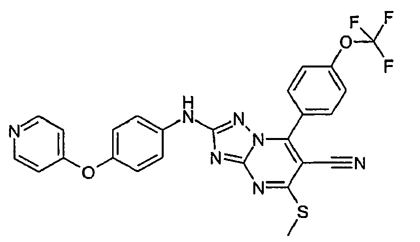 | 537 | |
| 107 | 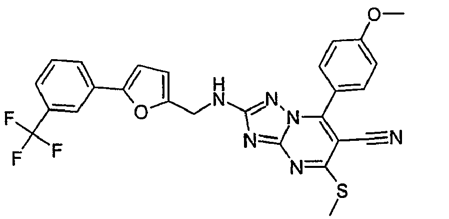 | 538 | |
| 108 | 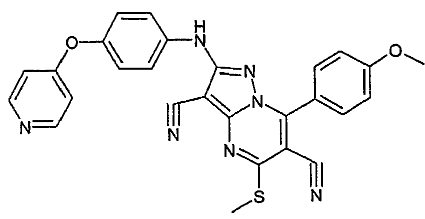 | 507 | |
| 109 | 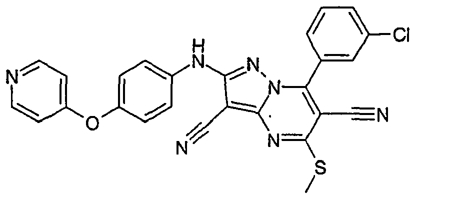 | 511 | |
| 110 | 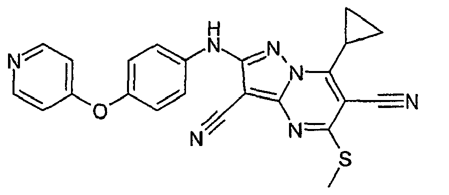 | 441 | |
| 111 | 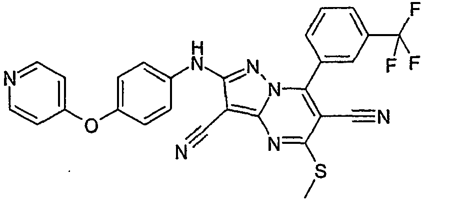 | 545 | |
| 112 | 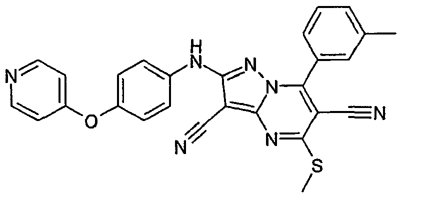 | 491 | |
| 113 | 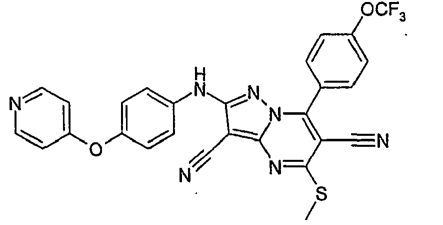 | 561 | |
| 114 | 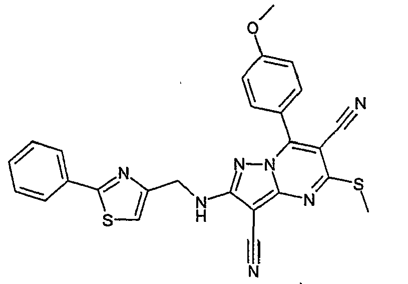 | 511 | |
| 115 | 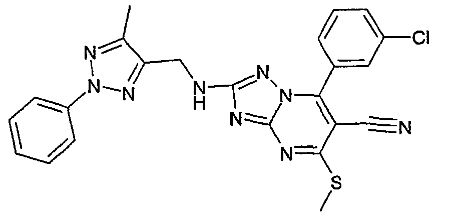 | 489 | |
| 116 | 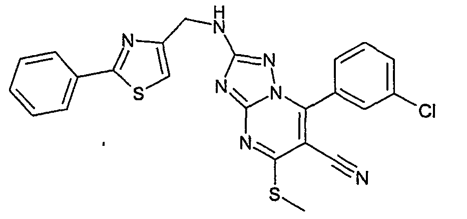 | 491 | |
| 117 | 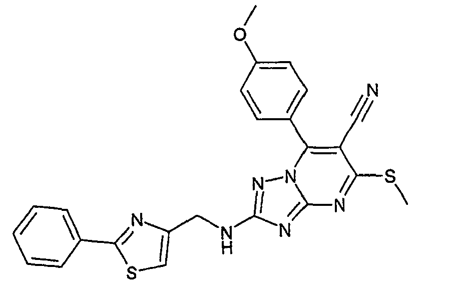 | 487 | |
| 118 | 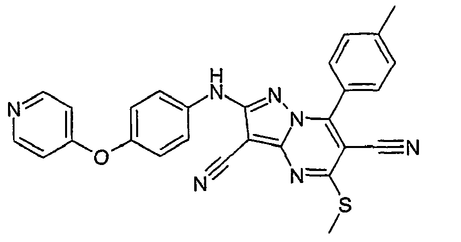 | 491 | |
| 119 | 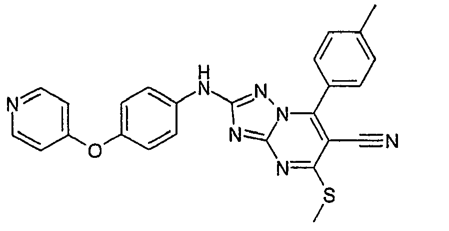 | 467 | |
| 120 | 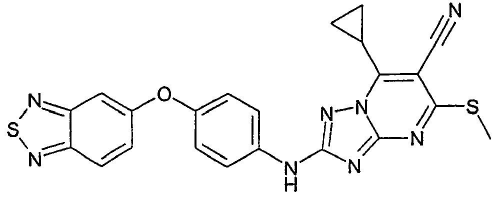 | 474 | |
| 121 | 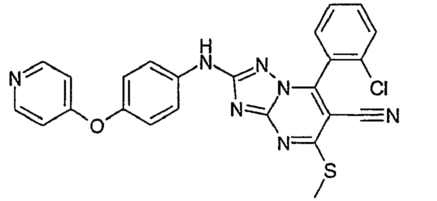 | 487 | |
| 122 | 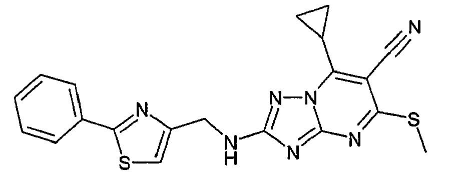 | 421 | |
| 123 | 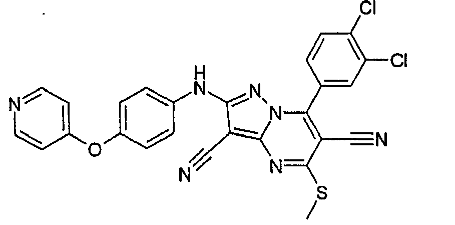 | 545 | |
| 124 | 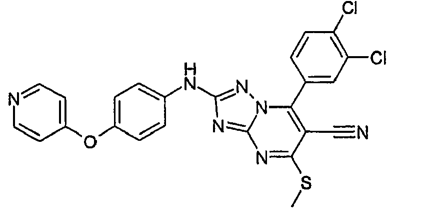 | 521 | |
| 125 | 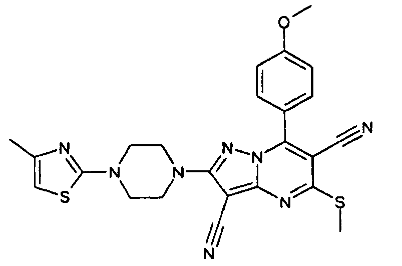 | 504 | |

### Beispiel 8

### Herstellung von 7-(3-Chlor-phenyl)-5-(4-methyl-piperazin-1-yl)-2-[4-(pyridin-4-yloxy)-phenylamino]-[1,2,4]triazolo[1,5-a]pyrimidin-6-carbonitril ("136")

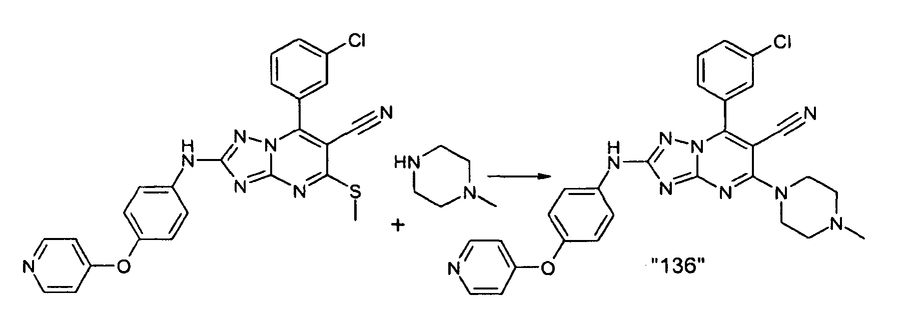

7-(3-Chlor-phenyl)-5-methylsulfanyl-2-[4-(pyridin-4-yloxy)-phenylamino]-[1,2,4]triazolo[1,5-a]pyrimidin-6-carbonitril (50 mg, 0.10 mol) wird in 1-Methylpiperazin (1 mL) 18 h in einem geschlossenen Gefäß auf 100°C erhitzt. Zu der abgekühlten Lösung wird Ethylacetat (5 mL) gegeben und der sich bildende Niederschlag abfiltriert, mit Diethylether gewaschen und getrocknet.
Man erhält 32 mg (0.06 mmol, 55 %) "136", [M+H]⁺ 539.

Analog erhält man die nachstehenden Verbindungen "137-139 "

| Nr. | Struktur | (M+H)⁺ | |
|---|---|---|---|
| 137 | 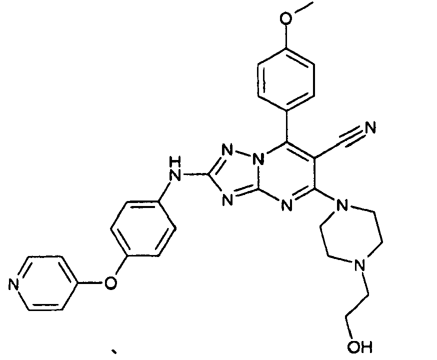 | 565 | |
| 138 | 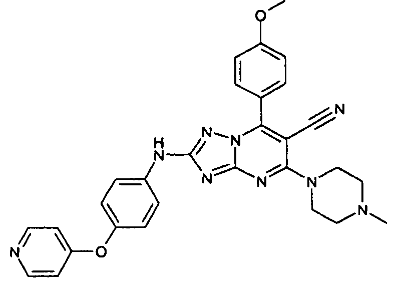 | 535 | |
| 139 | 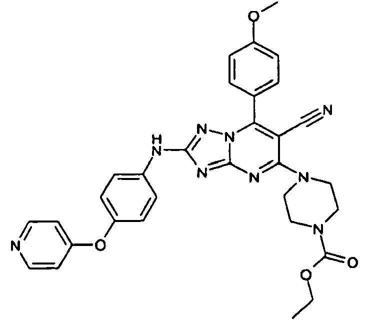 | 593 | |

Die nachfolgenden Beispiele betreffen Arzneimittel:

### Beispiel A: Injektionsgläser

Eine Lösung von 100 g eines Wirkstoffes der Formel I gemäß Anspruch 1 und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe 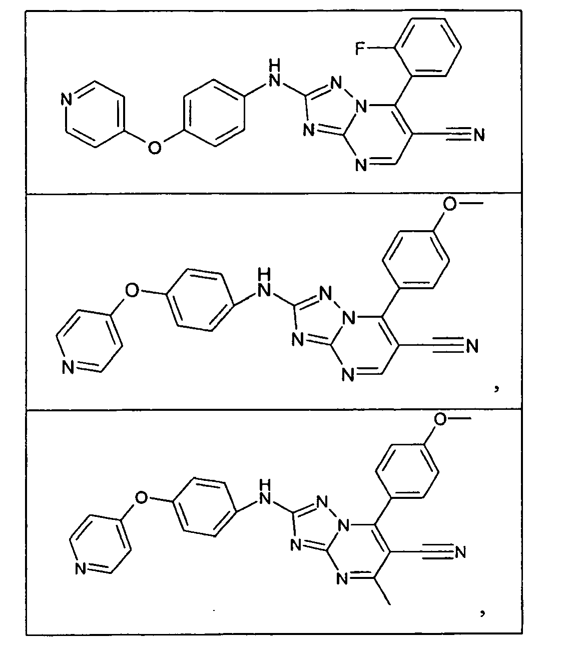 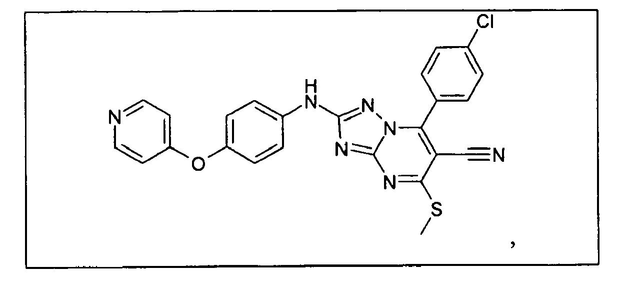 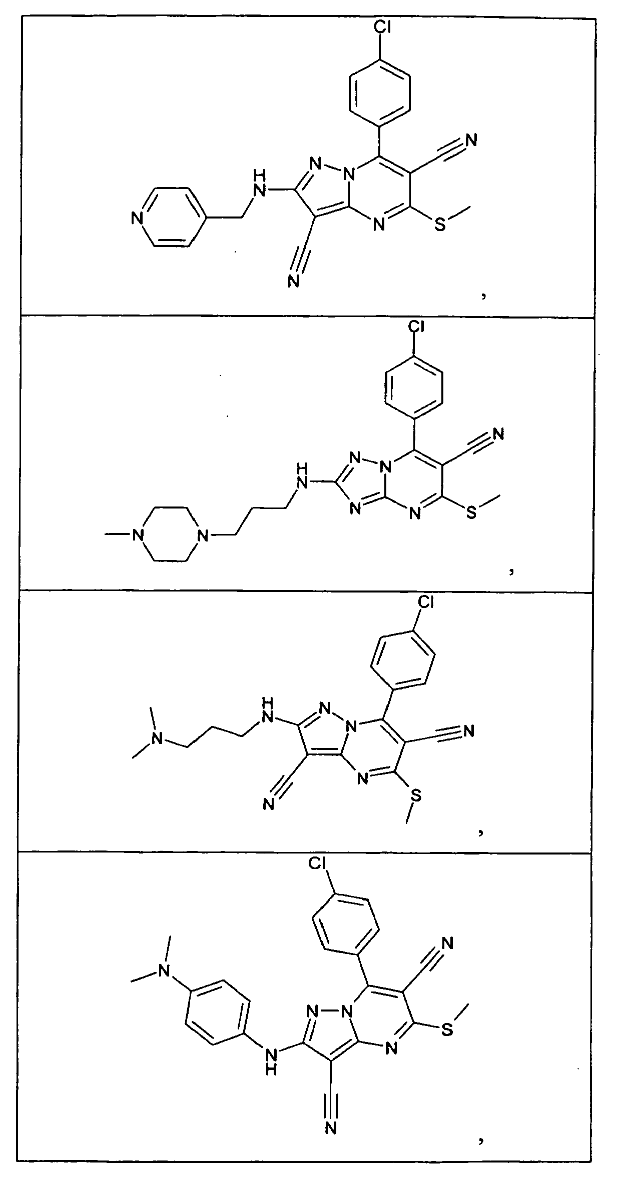 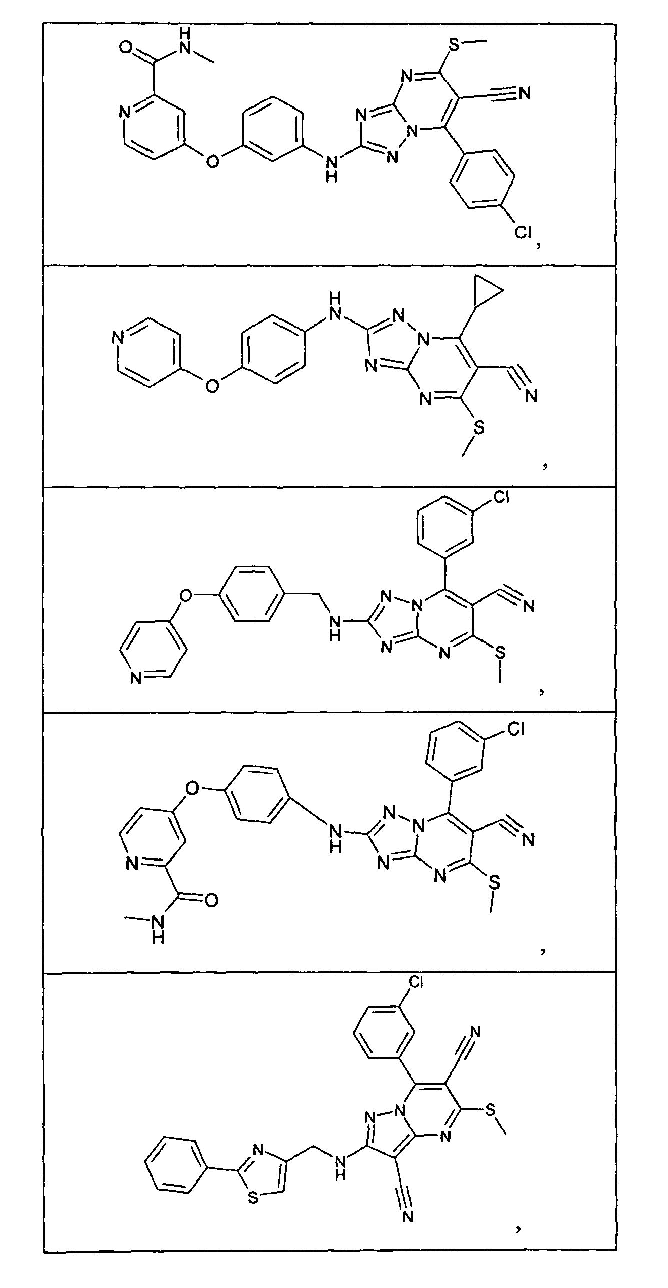 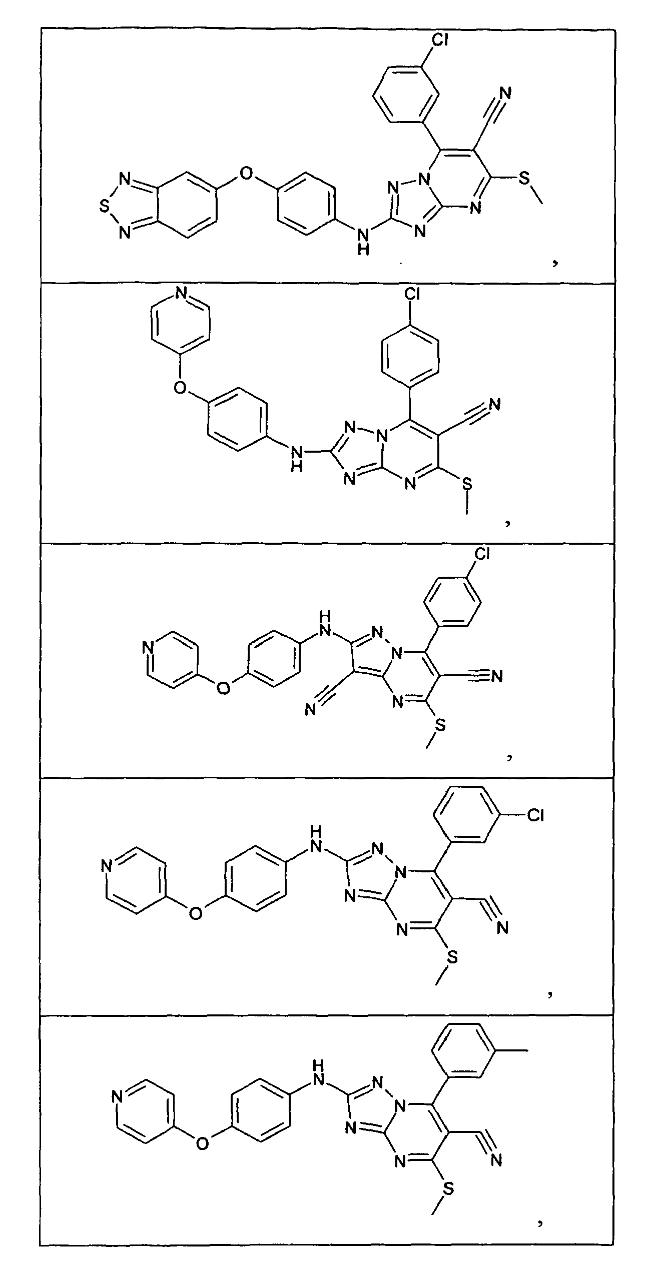 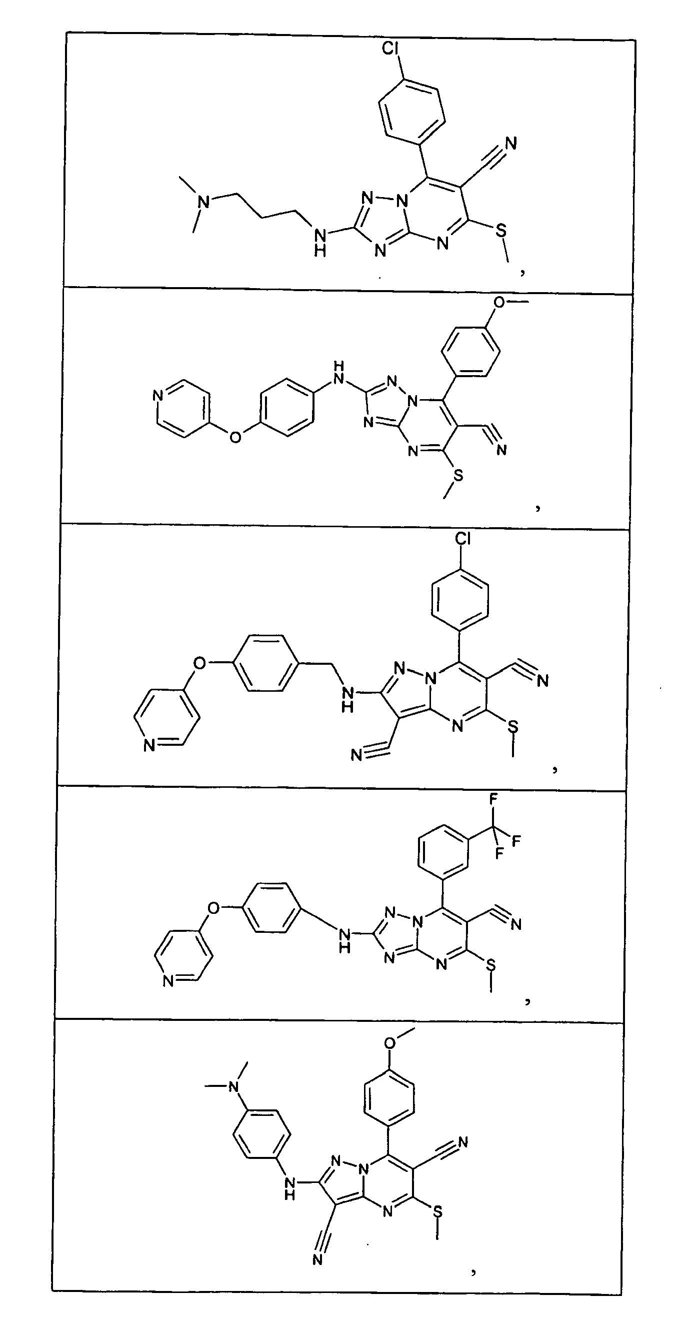 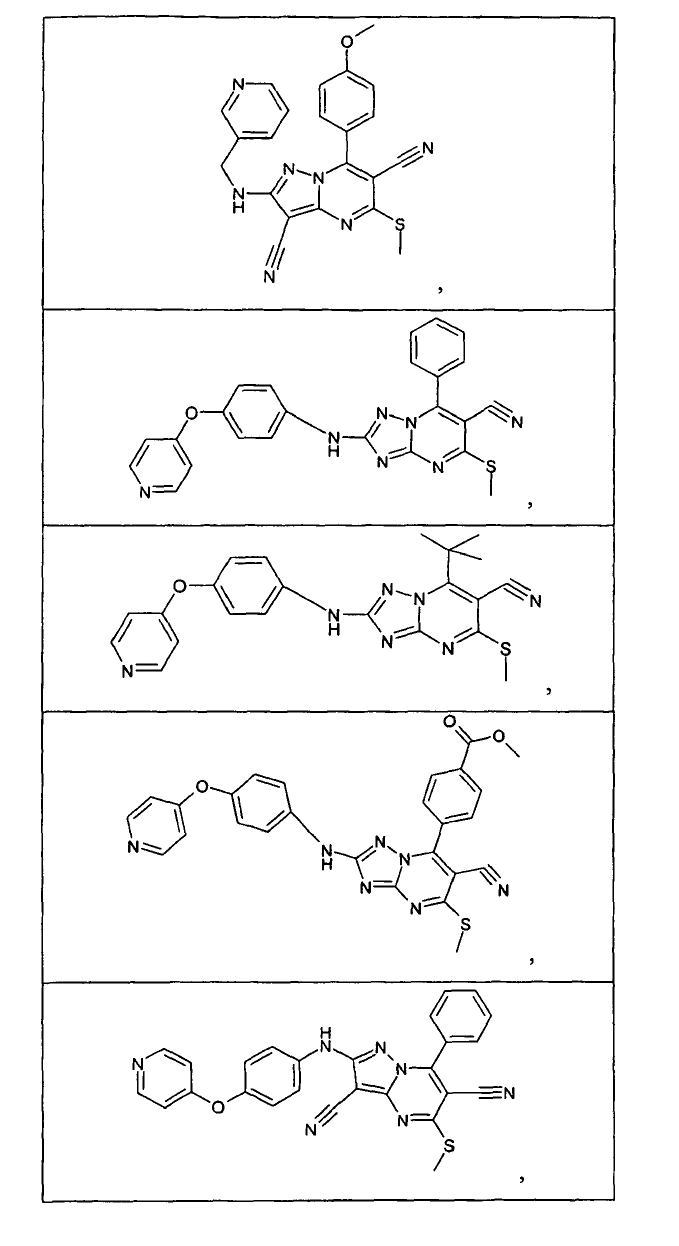 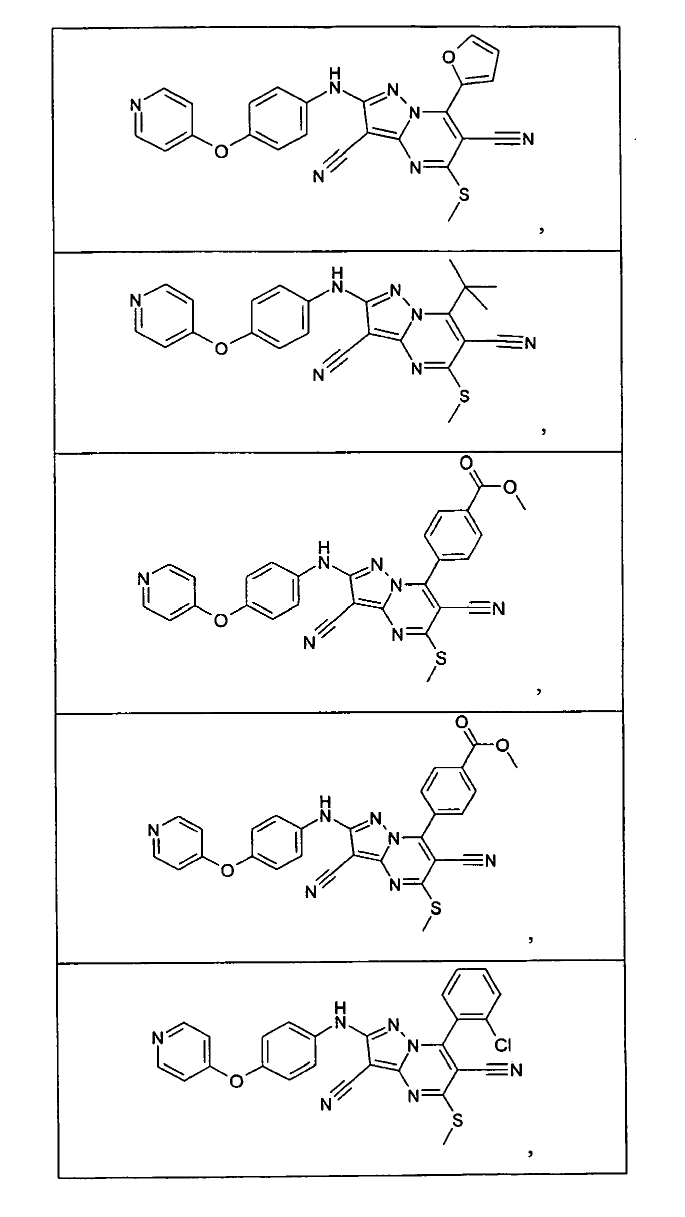 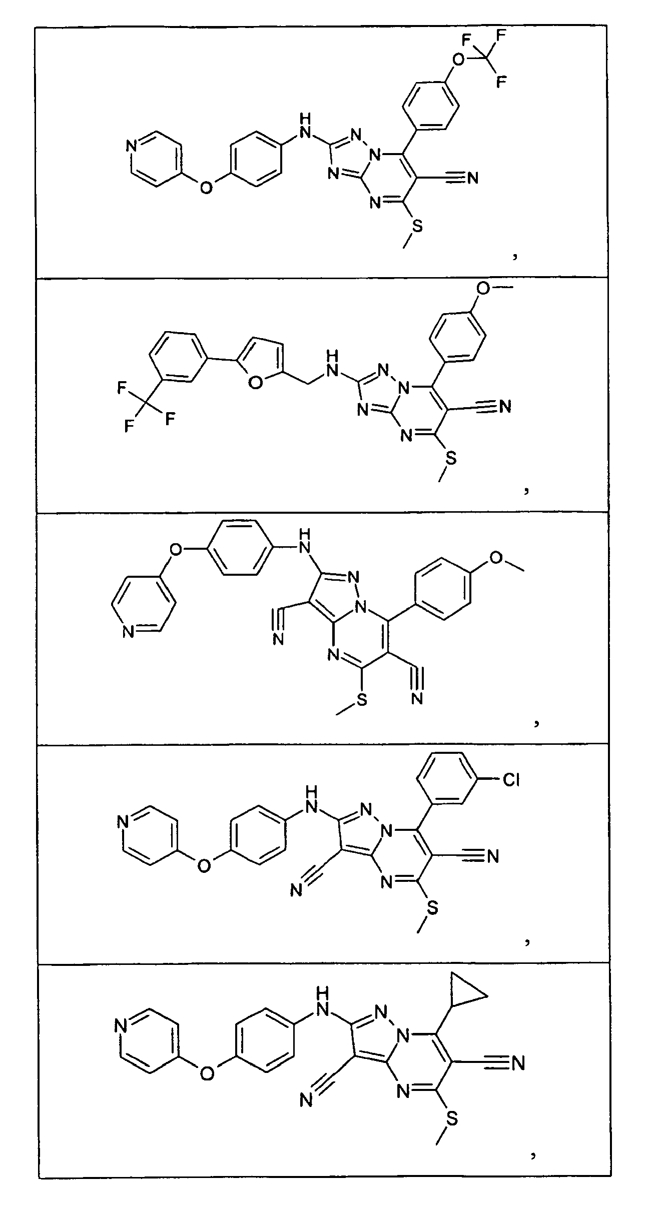 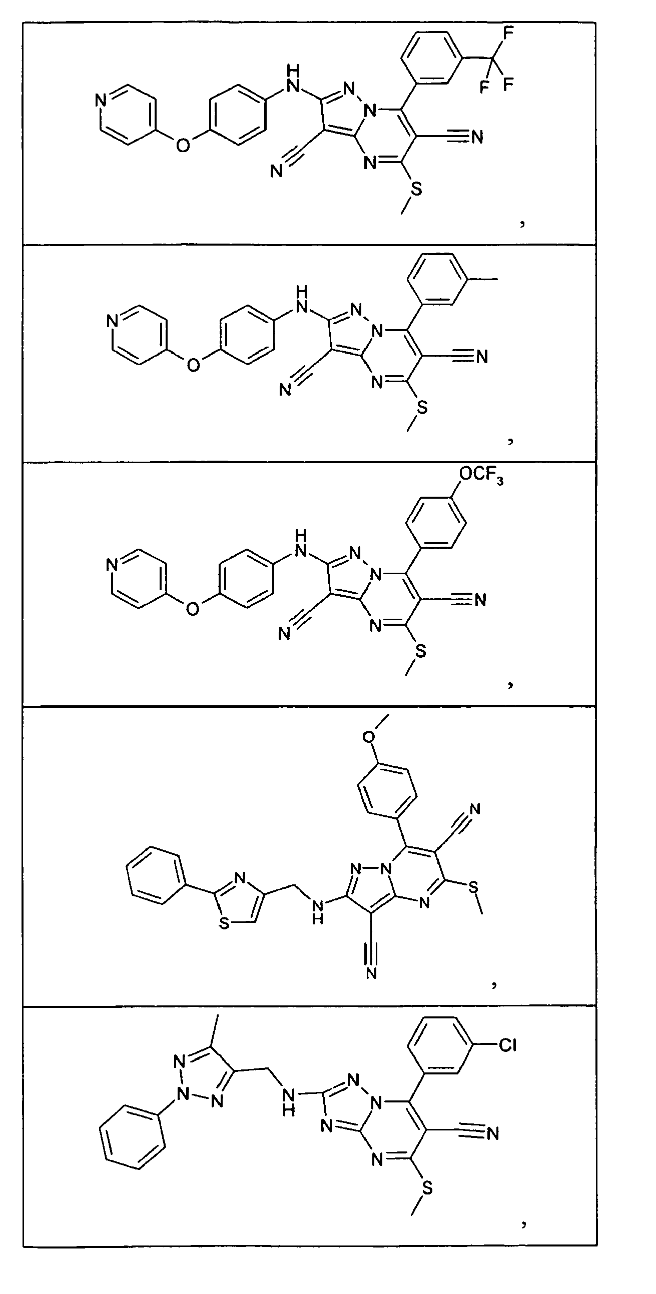 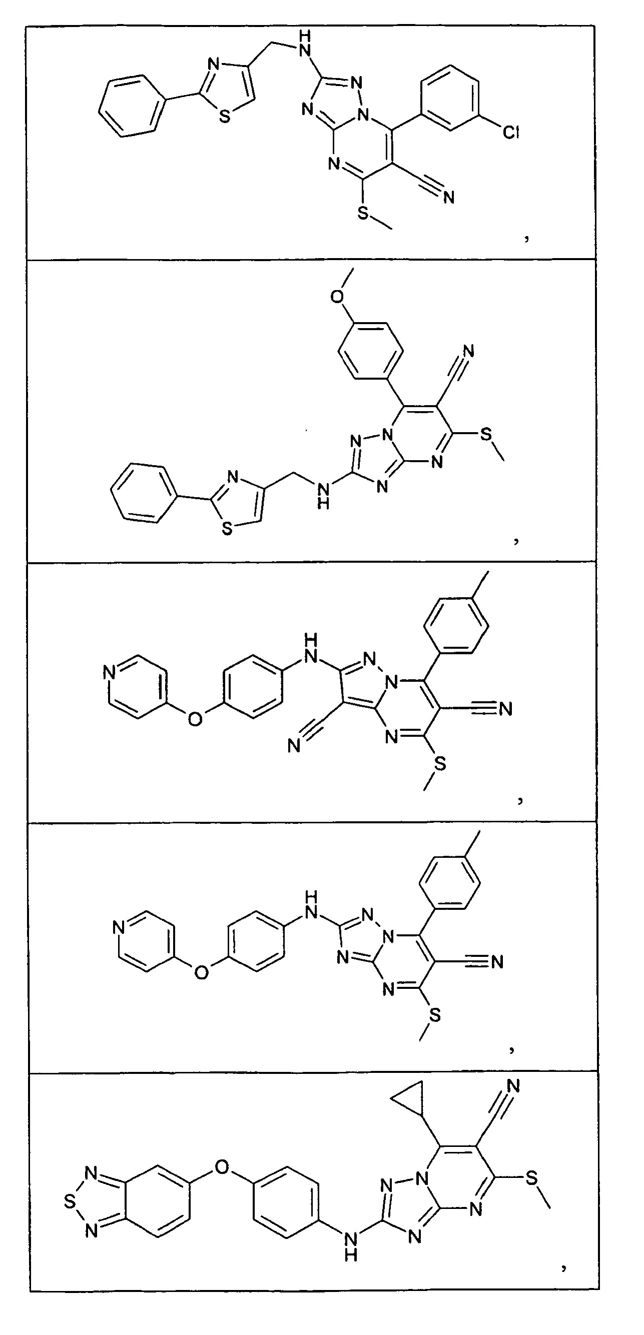 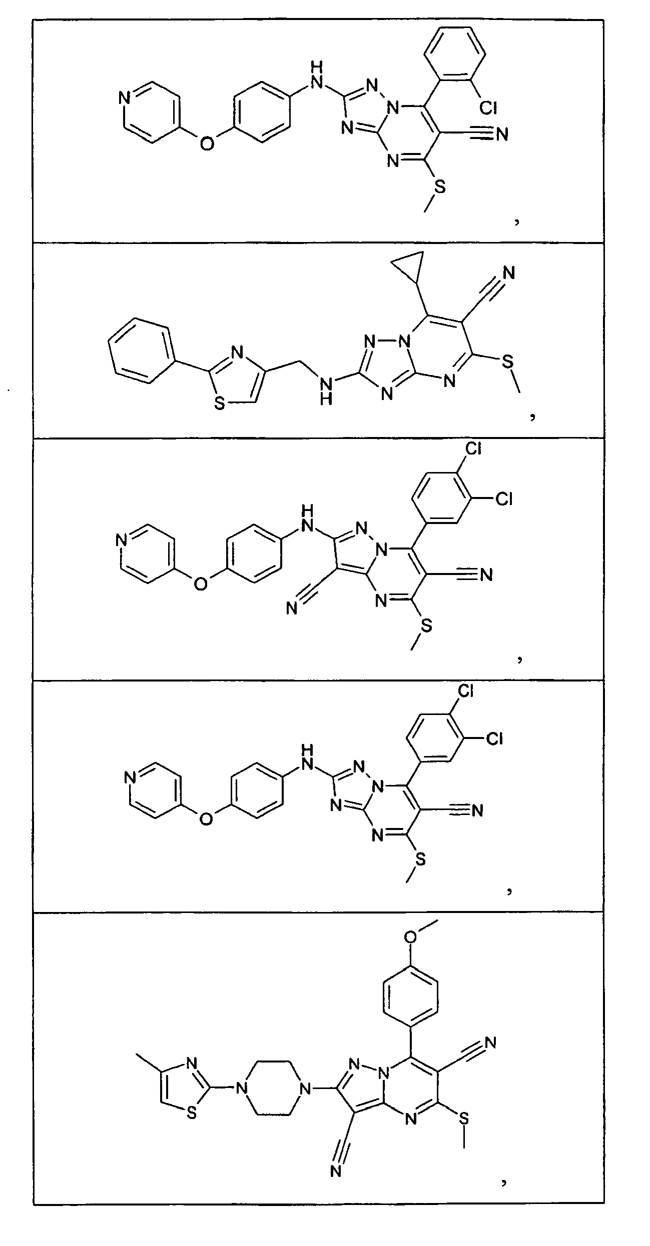 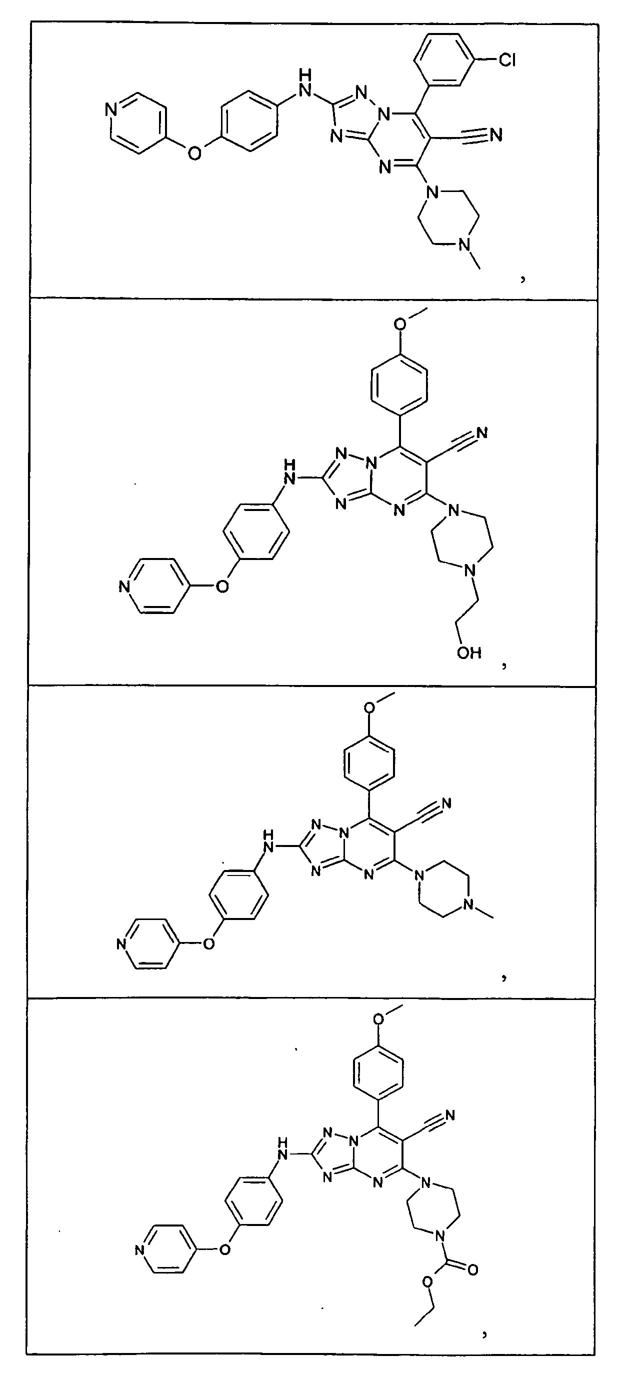 sowie ihre pharmazeutisch verwendbaren Solvate, Tautomere, Salze und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Solvate, Tautomere und Stereoisomeren, einschließlich deren Mischungen in allen Verhältnissen, sowie gegebenenfalls Träger- und/oder Hilfsstoffe.

3. Verwendung von Verbindungen nach Anspruch 1 sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung von Krankheiten,wobei die zu behandelnde Krankheit ein fester Tumor ist.

4. Verwendung nach Anspruch 3, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge stammt.

5. Verwendung nach Anspruch 3, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

6. Verwendung nach Anspruch 3, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

7. Verwendung von Verbindungen nach Anspruch 1 sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung von Krankheiten, wobei die zu behandelnde Krankheit ein Tumor des Blut- und Immunsystems ist.

8. Verwendung nach Anspruch 7, wobei der Tumor aus der Gruppe der akuten myelotischen Leukämie, der chronischen myelotischen Leukämie, akuten lymphatischen Leukämie und/oder chronischen lymphatischen Leukämie stammt.

9. Verwendung von Verbindungen nach Anspruch 1 sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung von Krankheiten an denen Angiogenese beteiligt ist.

10. Verwendung nach Anspruch 9, wobei es sich bei der Krankheit um eine Augenkrankheit handelt.

11. Verwendung von Verbindungen nach Anspruch 1 sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung von Krankheiten, ausgewählt aus der Gruppe
Retina-Vaskularisierung, diabetischer Retinopathie, altersbedingter Makula-Degeneration, Entzündungskrankheiten.

12. Verwendung nach Anspruch 11, wobei die Entzündungskrankheit aus der Gruppe rheumatoide Arthritis, Schuppenflechte, Kontaktdermatitis und Spät-Typ der Überempfindlichkeitsreaktion stammt.

13. Verwendung von Verbindungen nach Anspruch 1 sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung von Krankheiten, ausgewählt aus der Gruppe Osteosarkom, Osteoarthritis, Rachitis.

14. Verwendung von Verbindungen gemäß Anspruch 1 und/oder ihrer physiologisch unbedenklichen Salze und Solvate zur Herstellung eines Arzneimittels zur Behandlung von festen Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

15. Verwendung von Verbindungen gemäß Anspruch 1 und/oder ihrer physiologisch unbedenklichen Salze und Solvate zur Herstellung eines Arzneimittels zur Behandlung von festen Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

1. Compounds selected from the group 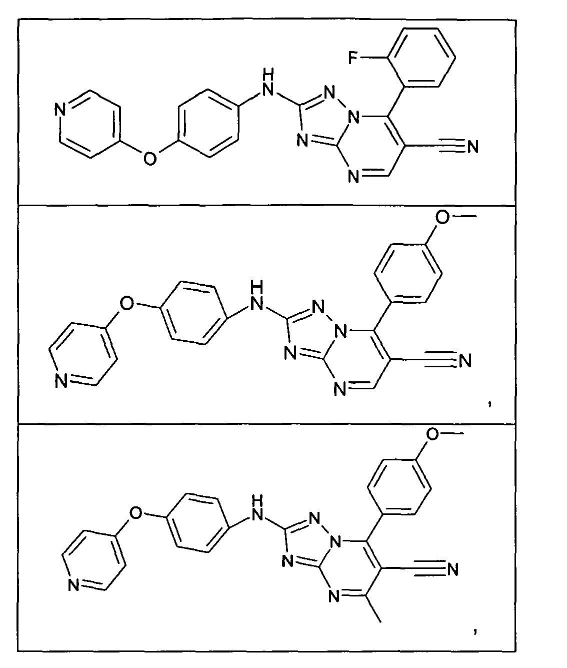 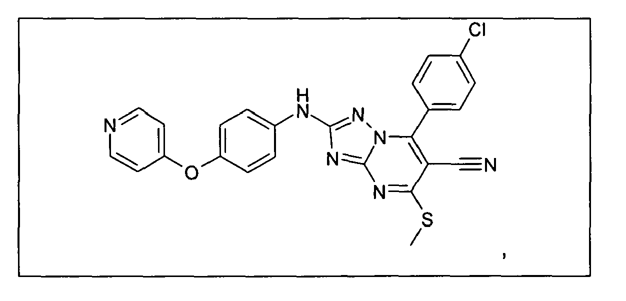 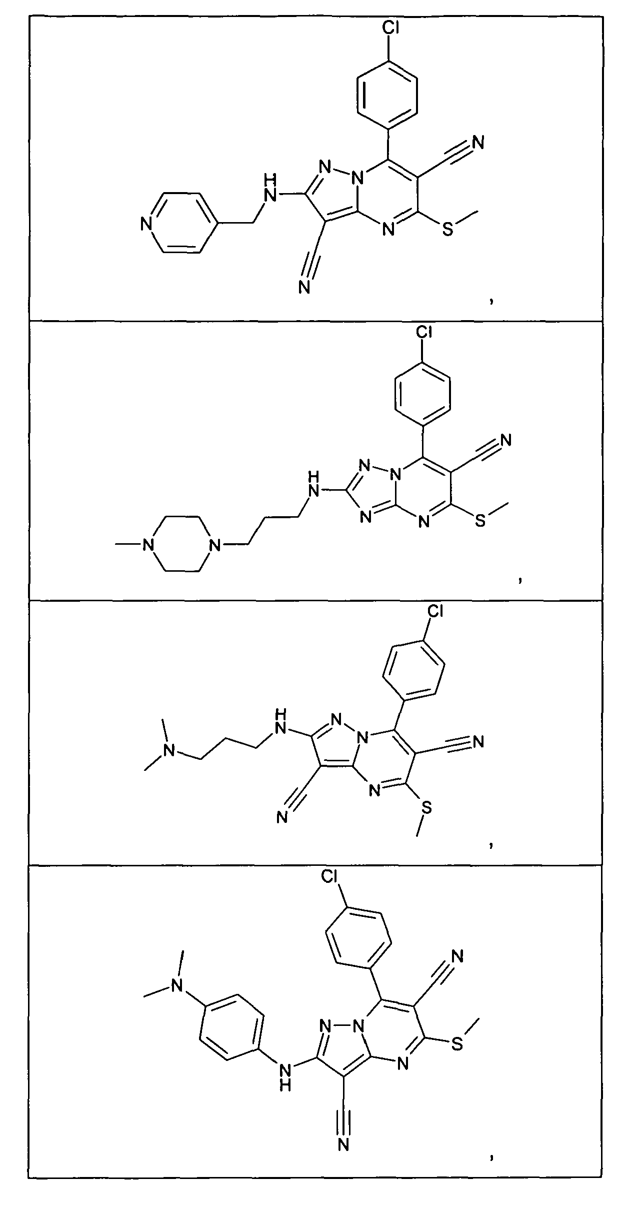 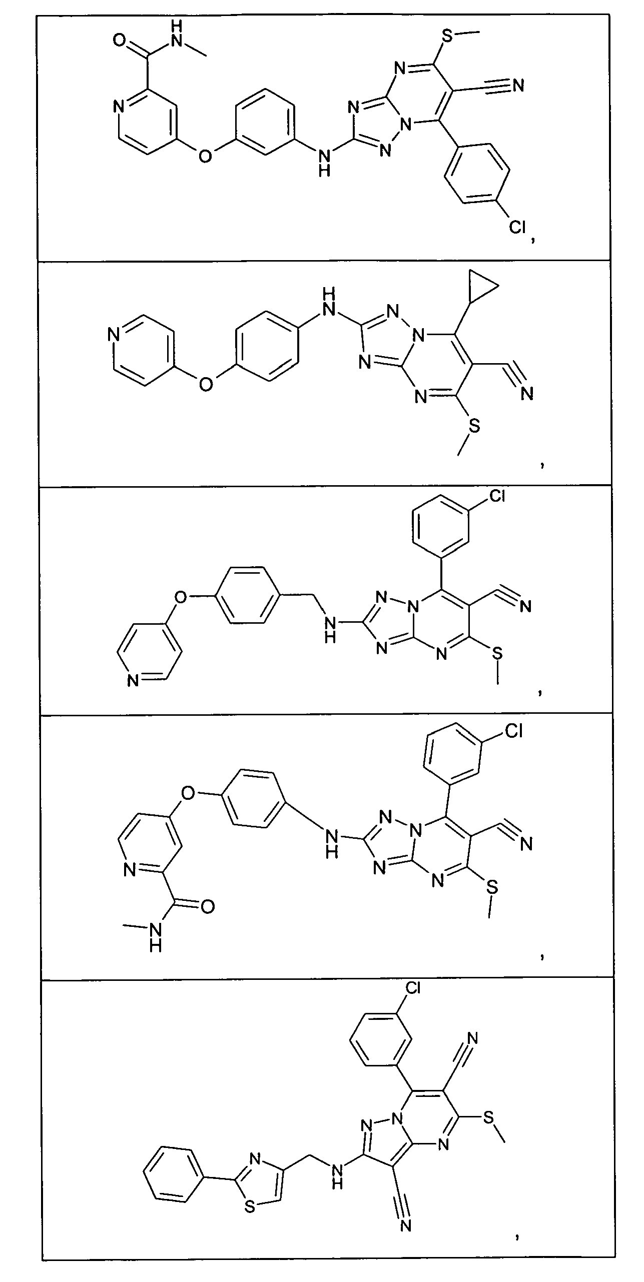 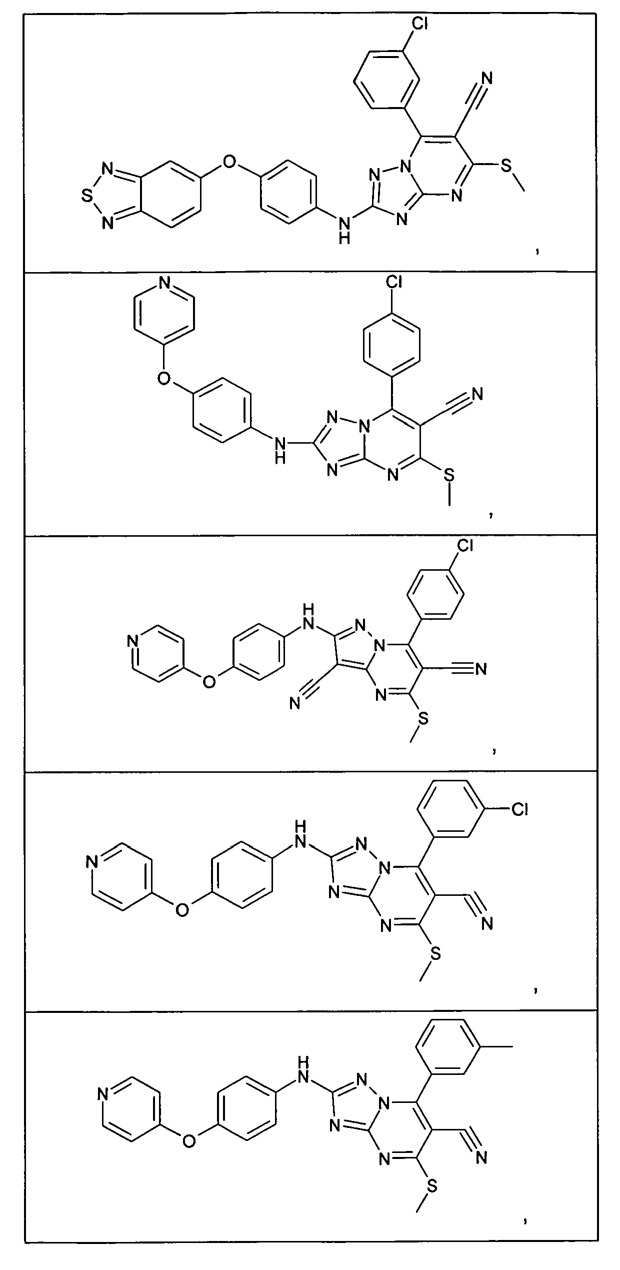 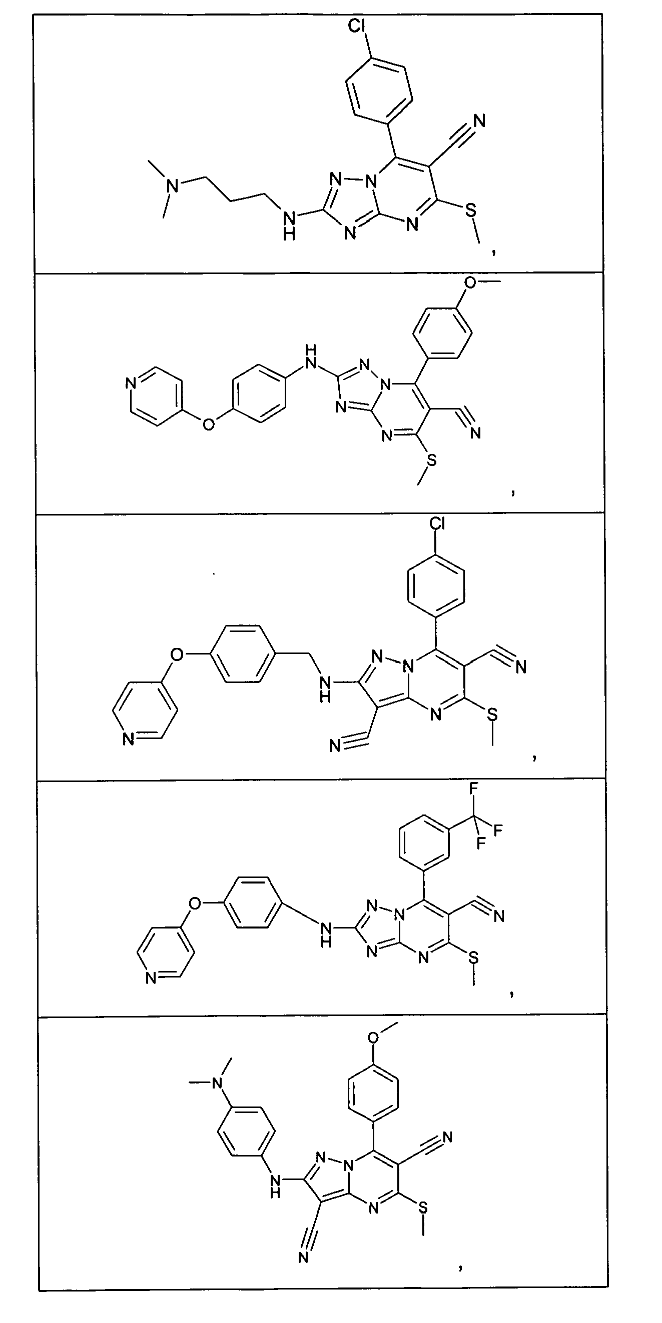 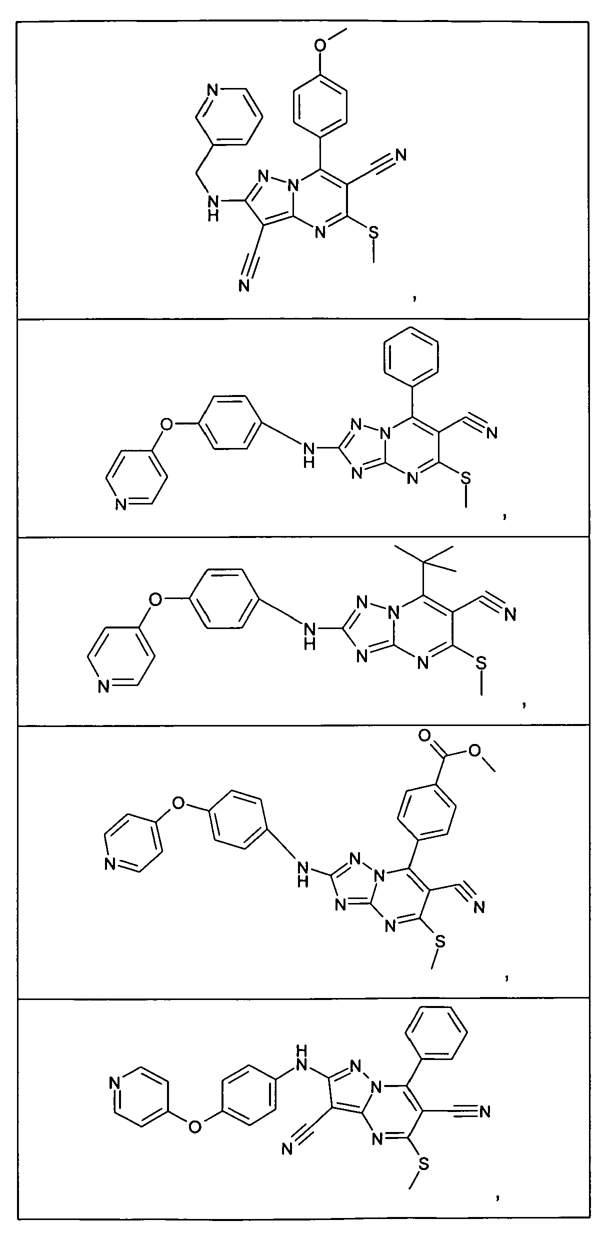 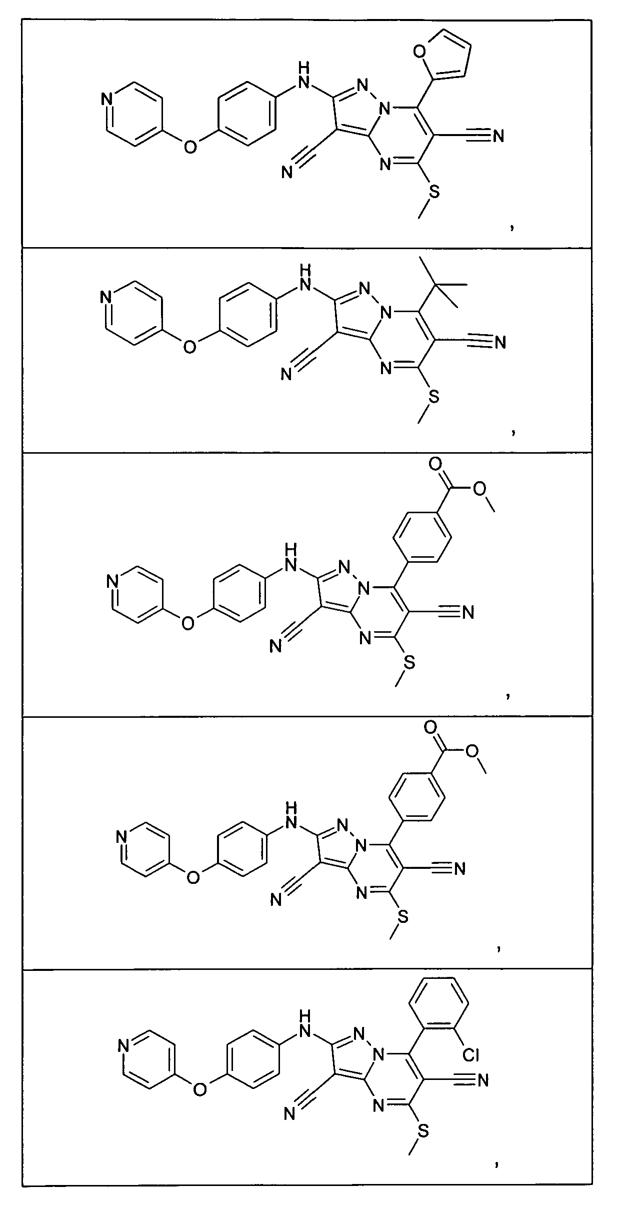 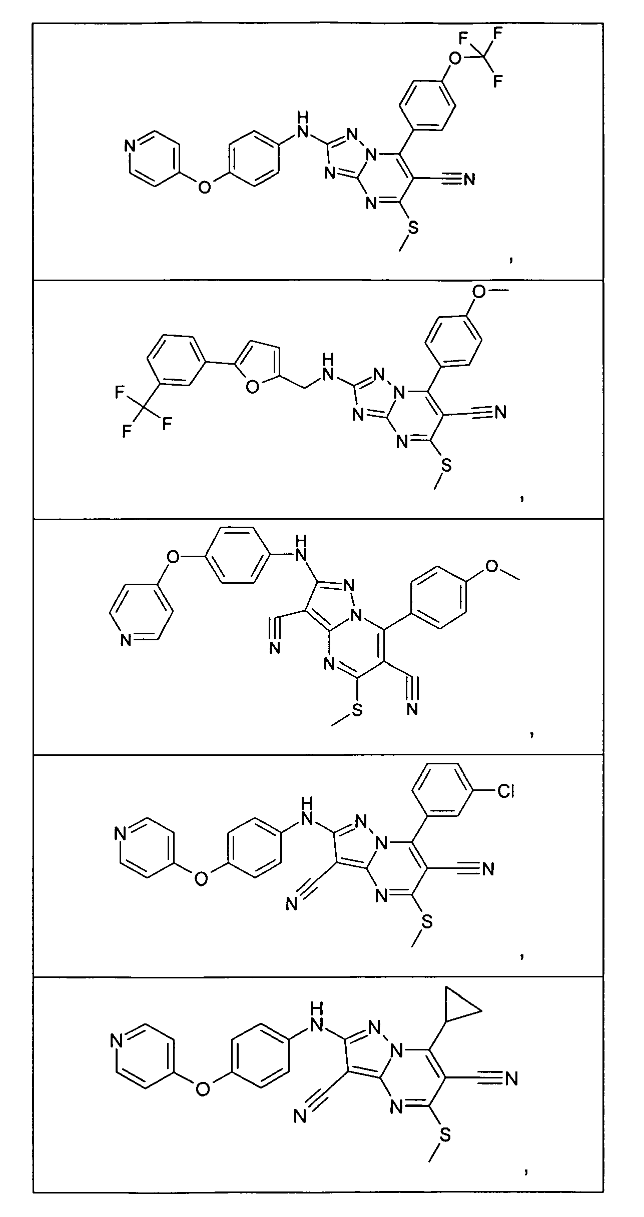 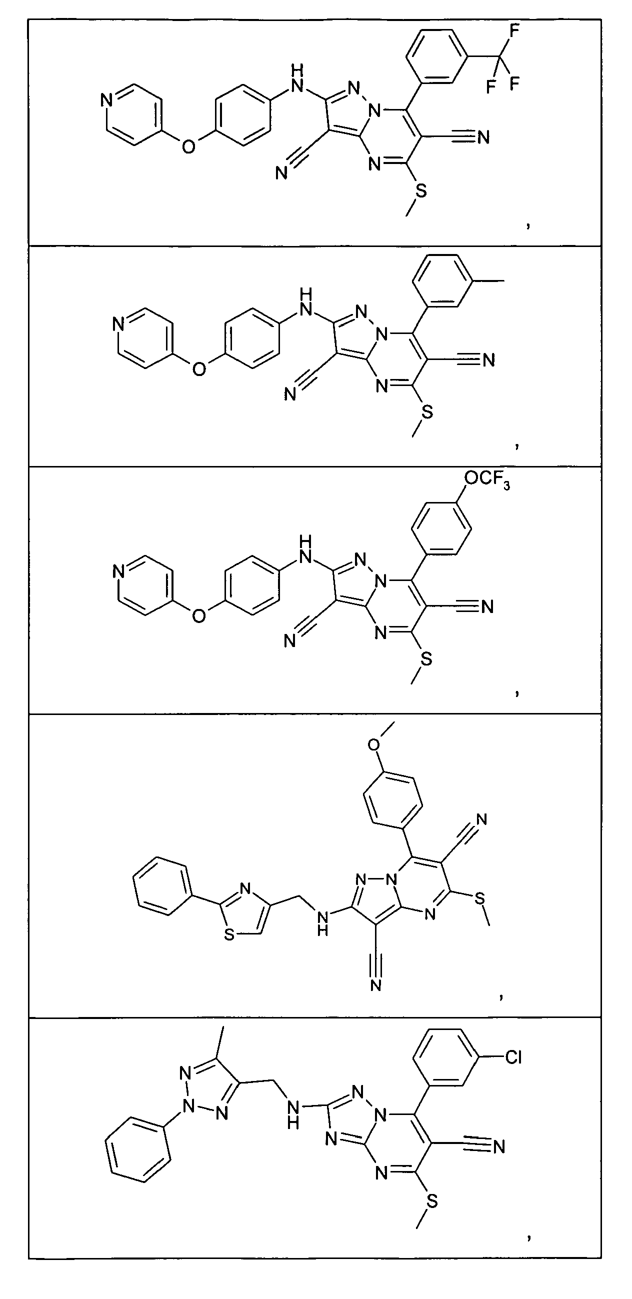 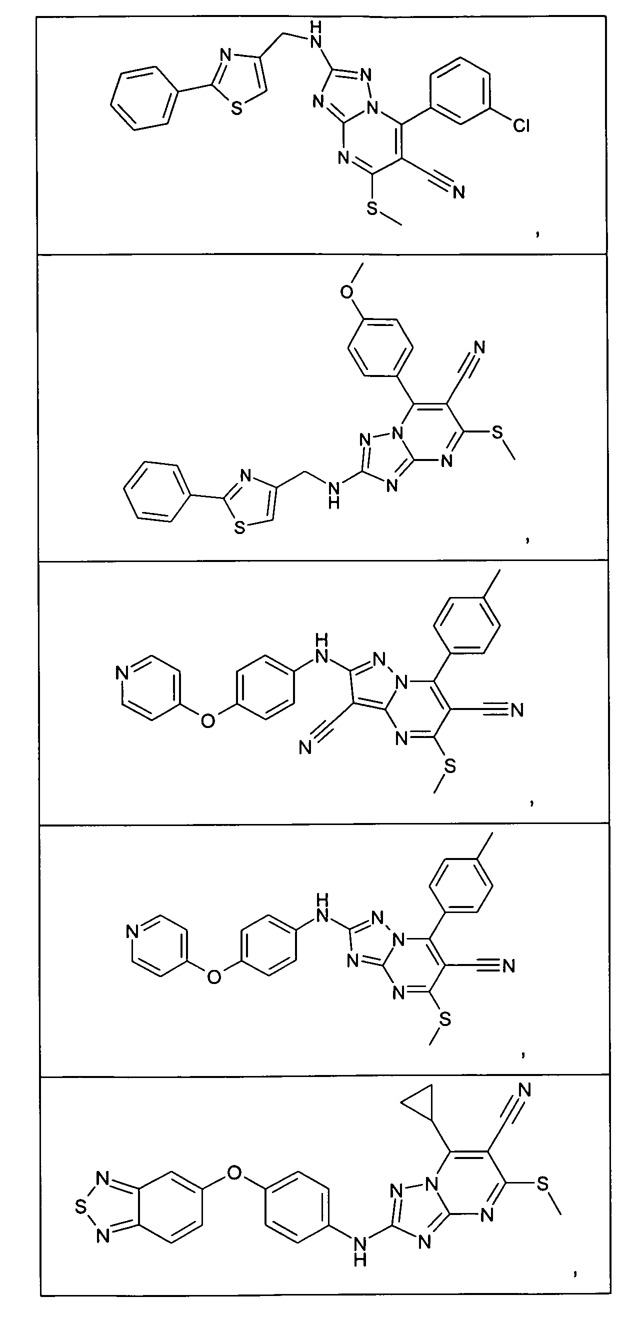 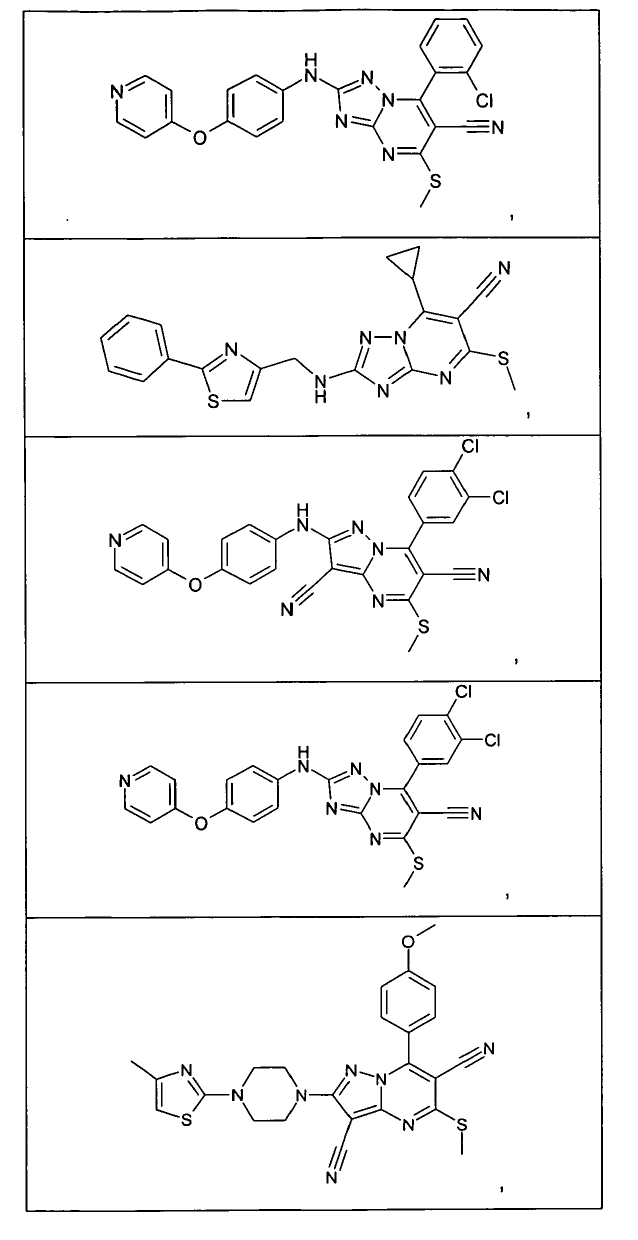 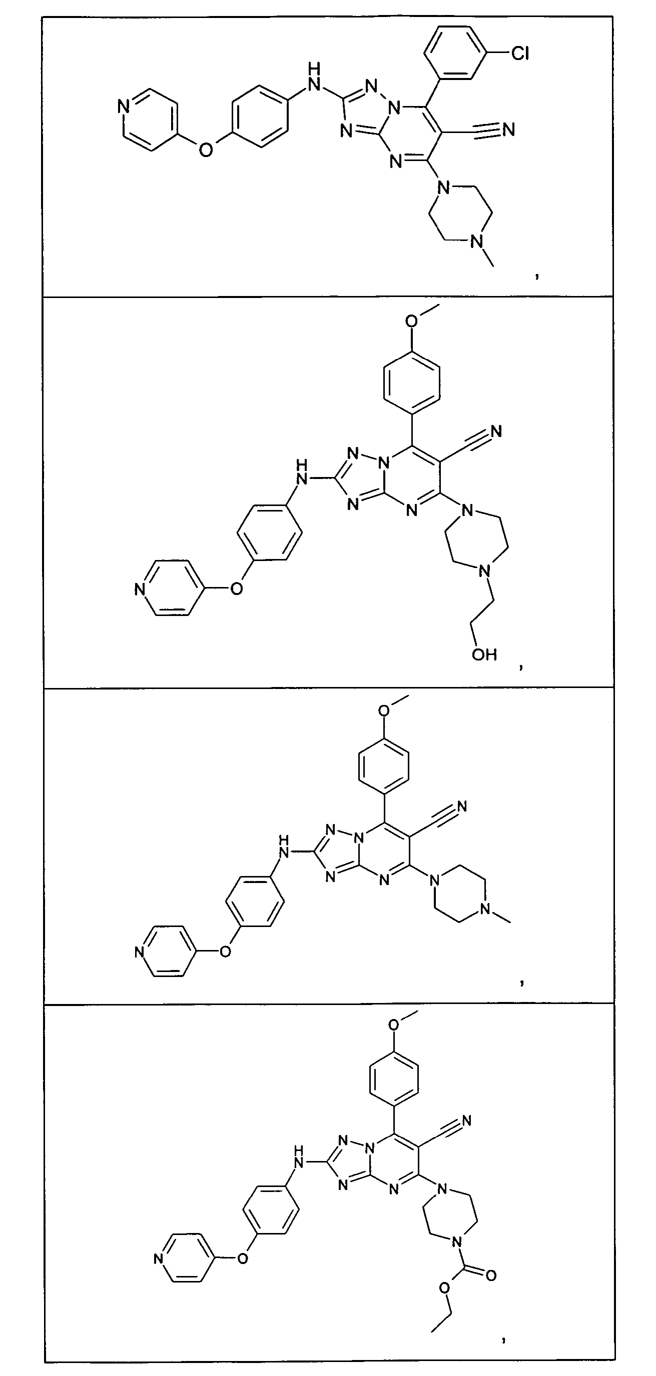 and pharmaceutically usable solvates, tautomers, salts and stereoisomers thereof, including mixtures thereof in all ratios.

2. Medicaments comprising at least one compound according to Claim 1 and/or pharmaceutically usable salts, solvates, tautomers and stereoisomers thereof, including mixtures thereof in all ratios, and optionally excipients and/or adjuvants.

3. Use of compounds according to Claim 1 and pharmaceutically usable salts, solvates, tautomers and stereoisomers thereof, including mixtures thereof in all ratios, for the preparation of a medicament for the treatment of diseases, where the disease to be treated is a solid tumour.

4. Use according to Claim 3, where the solid tumour originates from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

5. Use according to Claim 3, where the solid tumour originates from the group monocytic leukaemia, lung adenocarcinoma, small-cell lung carcinomas, pancreatic cancer, glioblastomas and breast carcinoma.

6. Use according to Claim 3, where the solid tumour originates from the group of lung adenocarcinoma, small-cell lung carcinomas, pancreatic cancer, glioblastomas, colon carcinoma and breast carcinoma.

7. Use of compounds according to Claim 1 and pharmaceutically usable salts, solvates, tautomers and stereoisomers thereof, including mixtures thereof in all ratios, for the preparation of a medicament for the treatment of diseases, where the disease to be treated is a tumour of the blood and immune system.

8. Use according to Claim 7, where the tumour originates from the group of acute myeloid leukaemia, chronic myeloid leukaemia, acute lymphatic leukaemia and/or chronic lymphatic leukaemia.

9. Use of compounds according to Claim 1 and pharmaceutically usable salts, solvates, tautomers and stereoisomers thereof, including mixtures thereof in all ratios, for the preparation of a medicament for the treatment of diseases in which angiogenesis is implicated.

10. Use according to Claim 9, where the disease is an eye disease.

11. Use of compounds according to Claim 1 and pharmaceutically usable salts, solvates, tautomers and stereoisomers thereof, including mixtures thereof in all ratios, for the preparation of a medicament for the treatment of diseases selected from the group retinal vascularisation, diabetic retinopathy, age-induced macular degeneration, inflammatory diseases.

12. Use according to Claim 11, where the inflammatory disease originates from the group rheumatoid arthritis, psoriasis, contact dermatitis and delayed hypersensitivity reaction.

13. Use of compounds according to Claim 1 and pharmaceutically usable salts, solvates, tautomers and stereoisomers thereof, including mixtures thereof in all ratios, for the preparation of a medicament for the treatment of diseases selected from the group osteosarcoma, osteoarthritis, rickets.

14. Use of compounds according to Claim 1 and/or physiologically acceptable salts and solvates thereof for the preparation of a medicament for the treatment of solid tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) another angiogenesis inhibitor.

15. Use of compounds according to Claim 1 and/or physiologically acceptable salts and solvates thereof for the preparation of a medicament for the treatment of solid tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) another angiogenesis inhibitor.

## Revendications

1. Composés choisis parmi le groupe 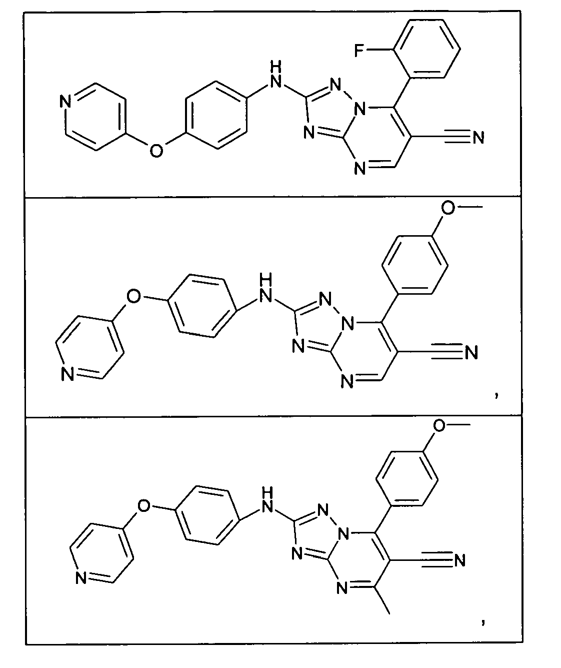 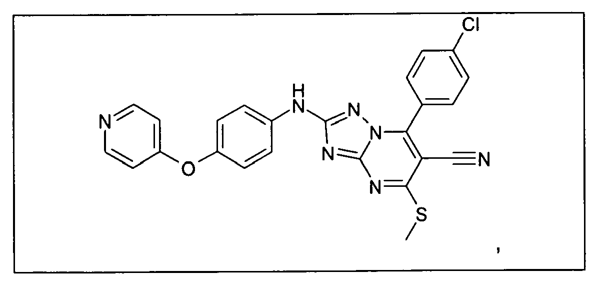 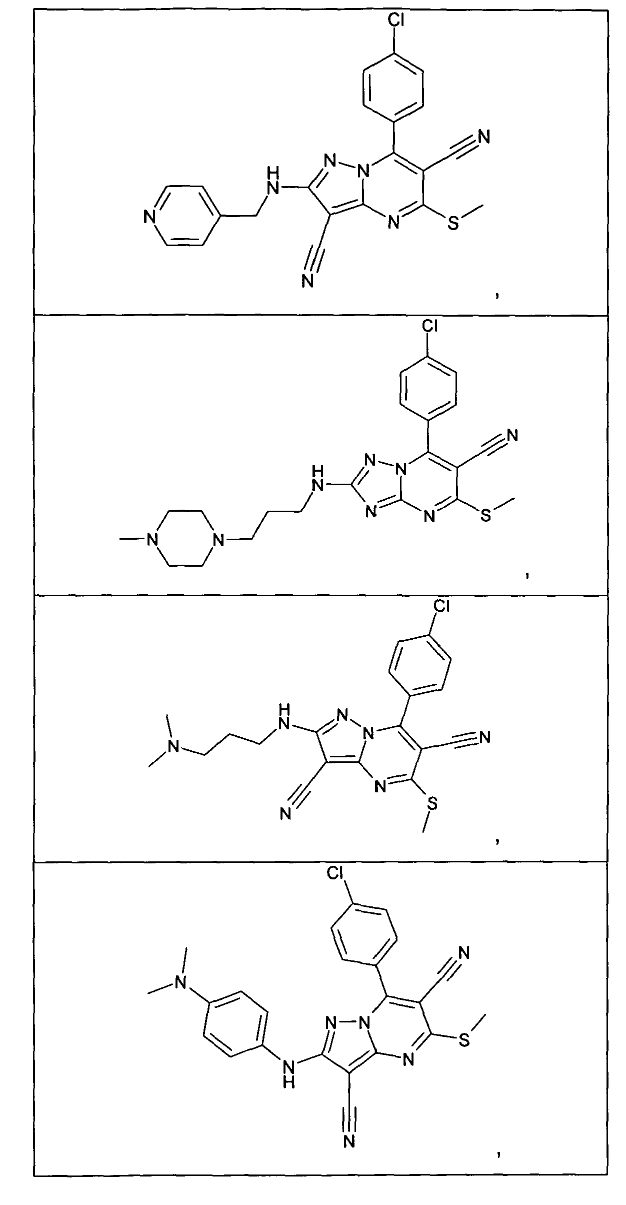 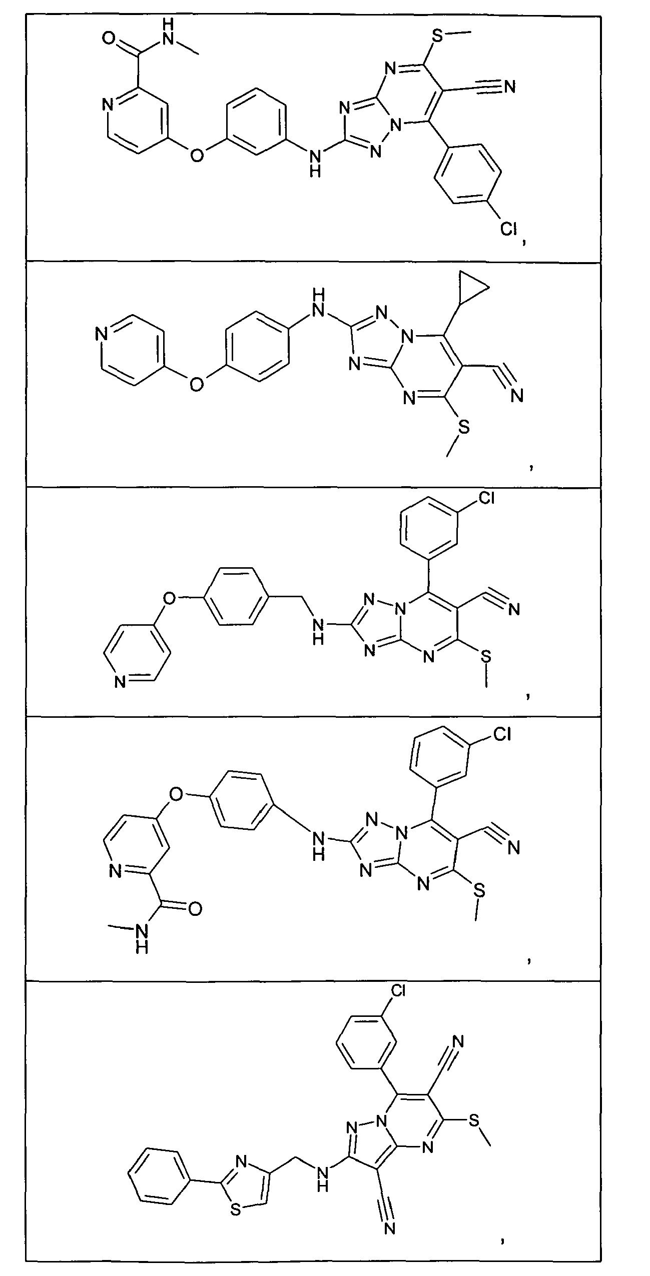 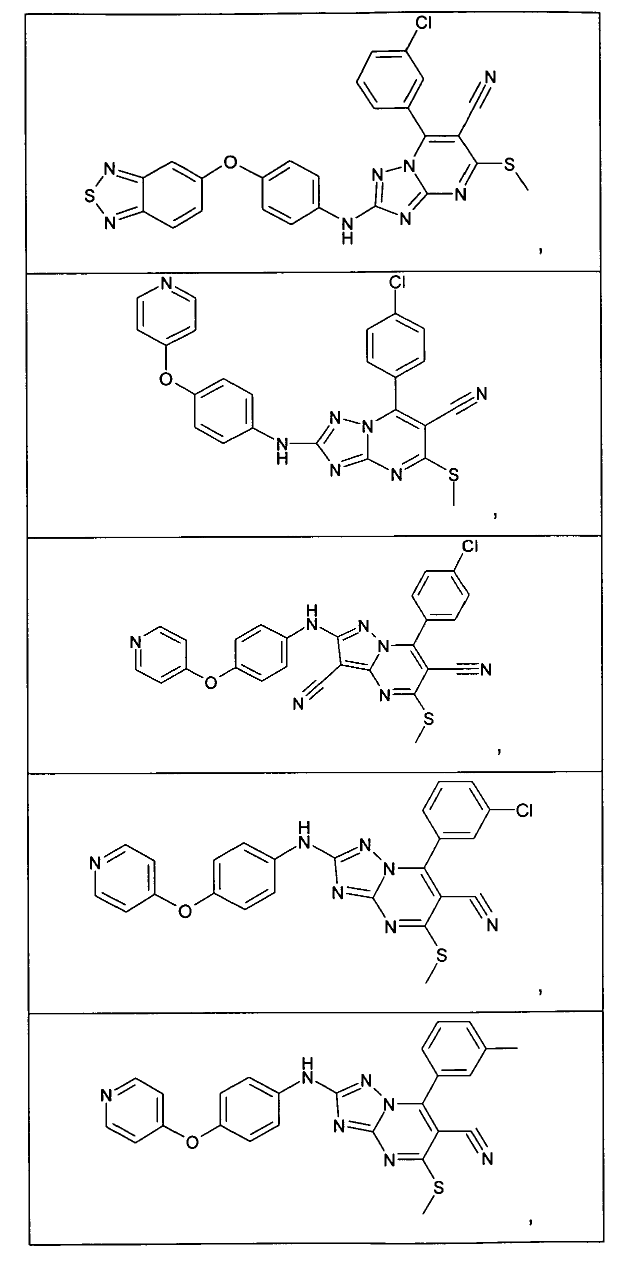 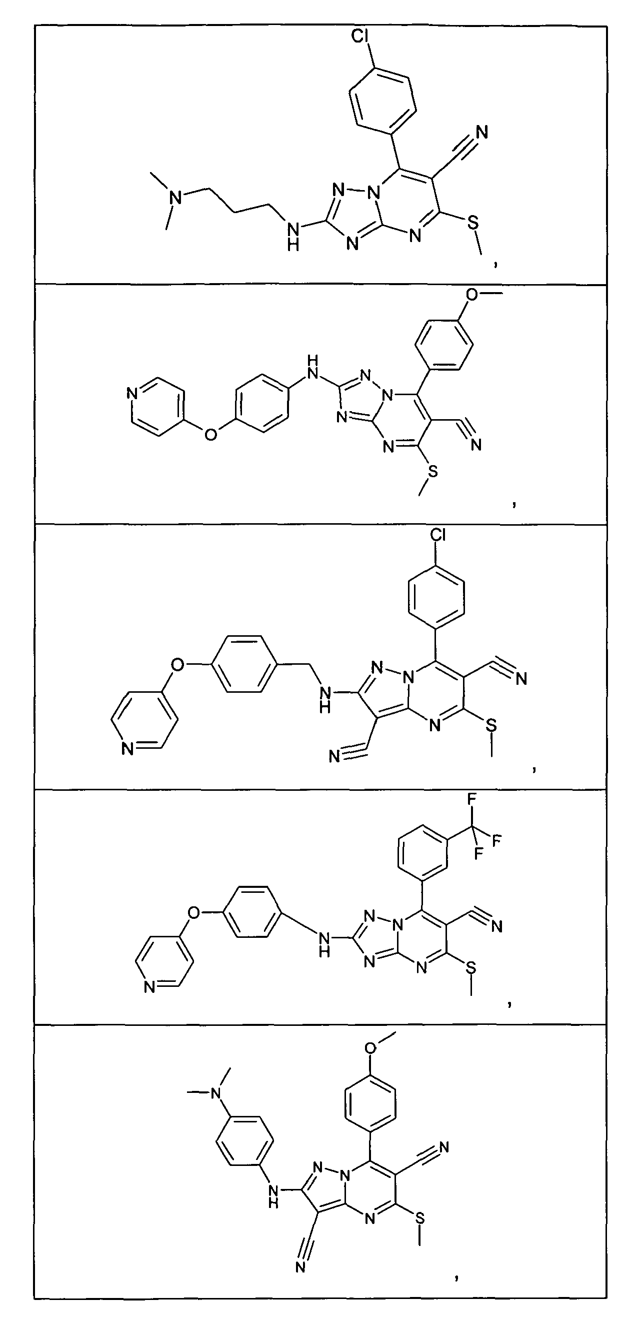 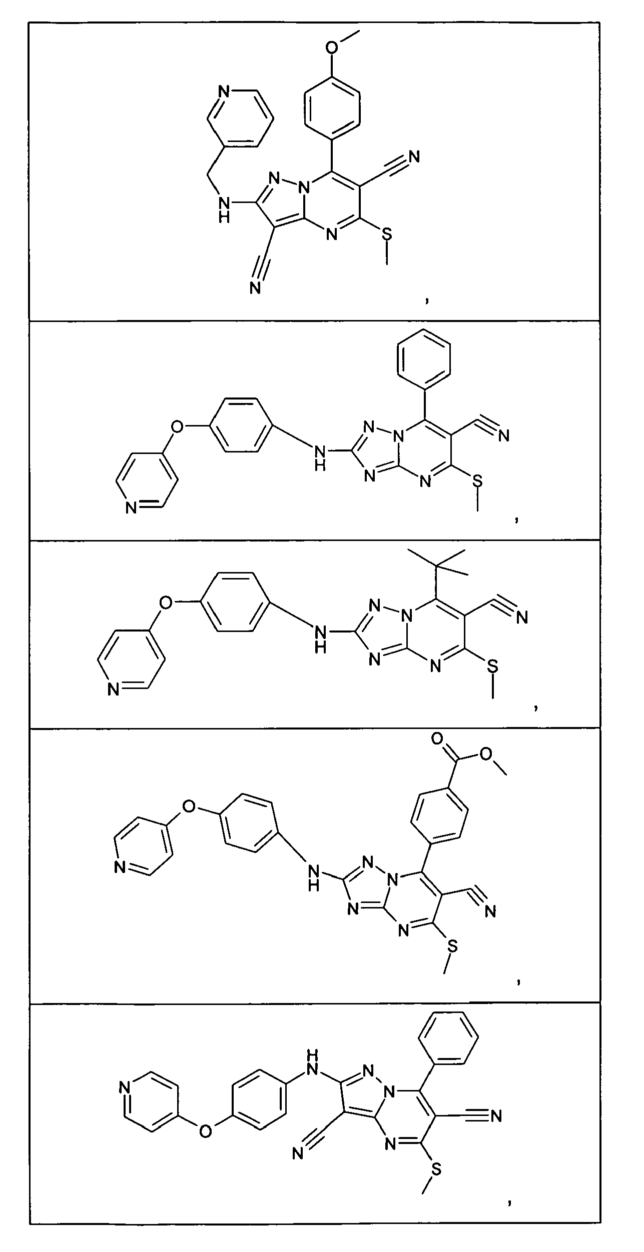 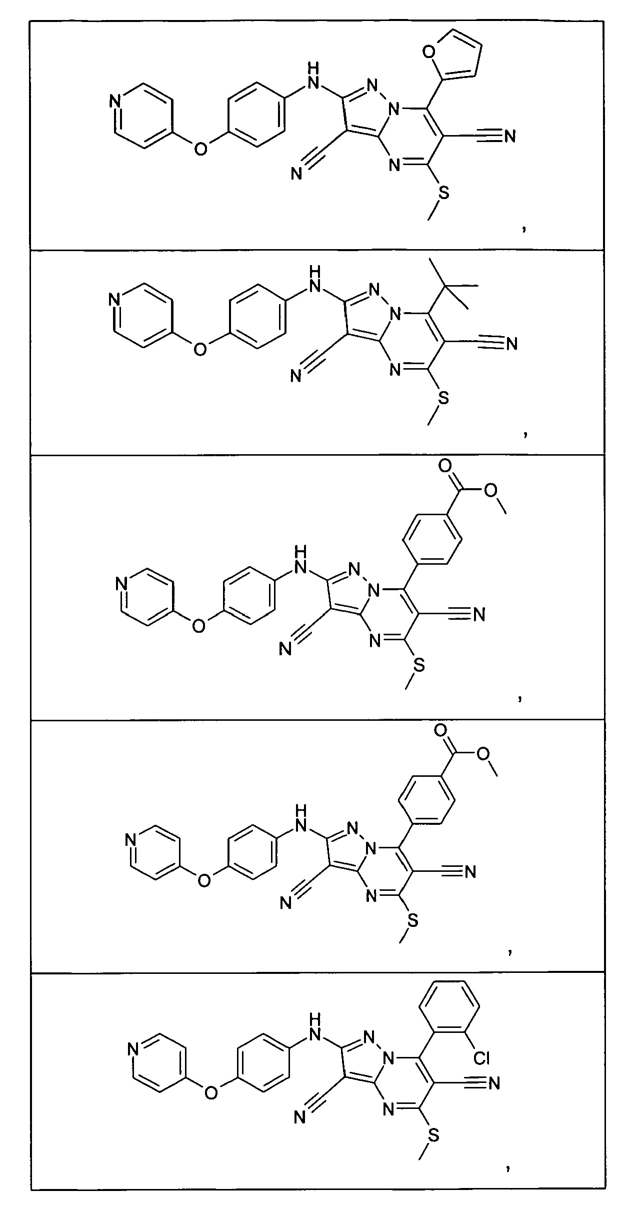 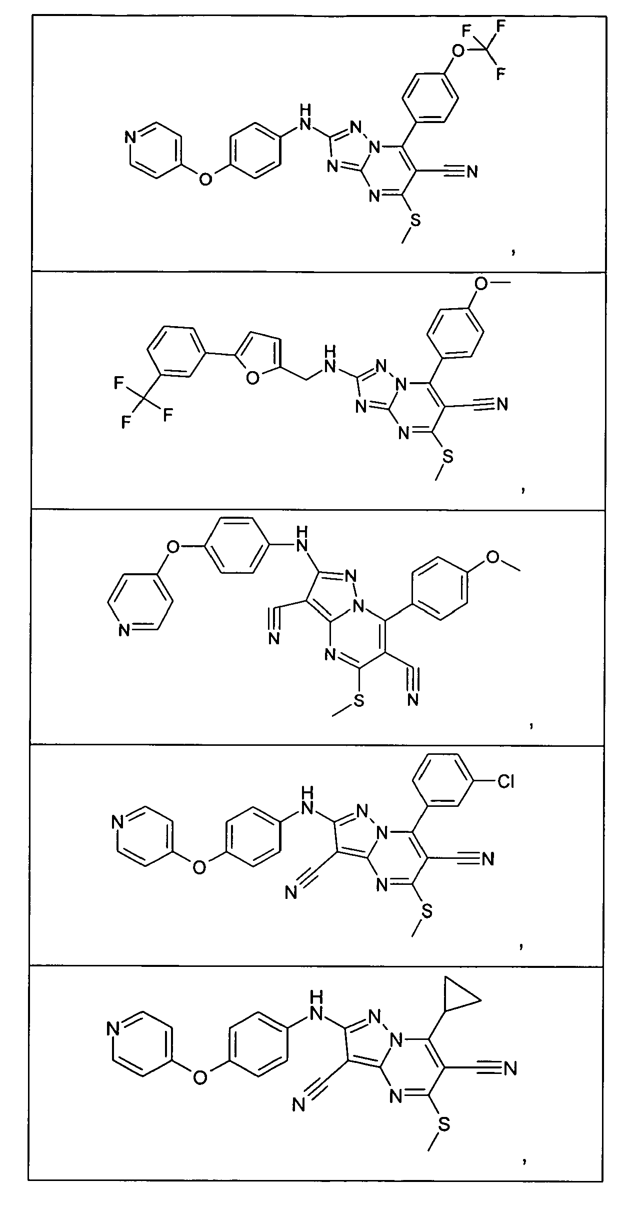 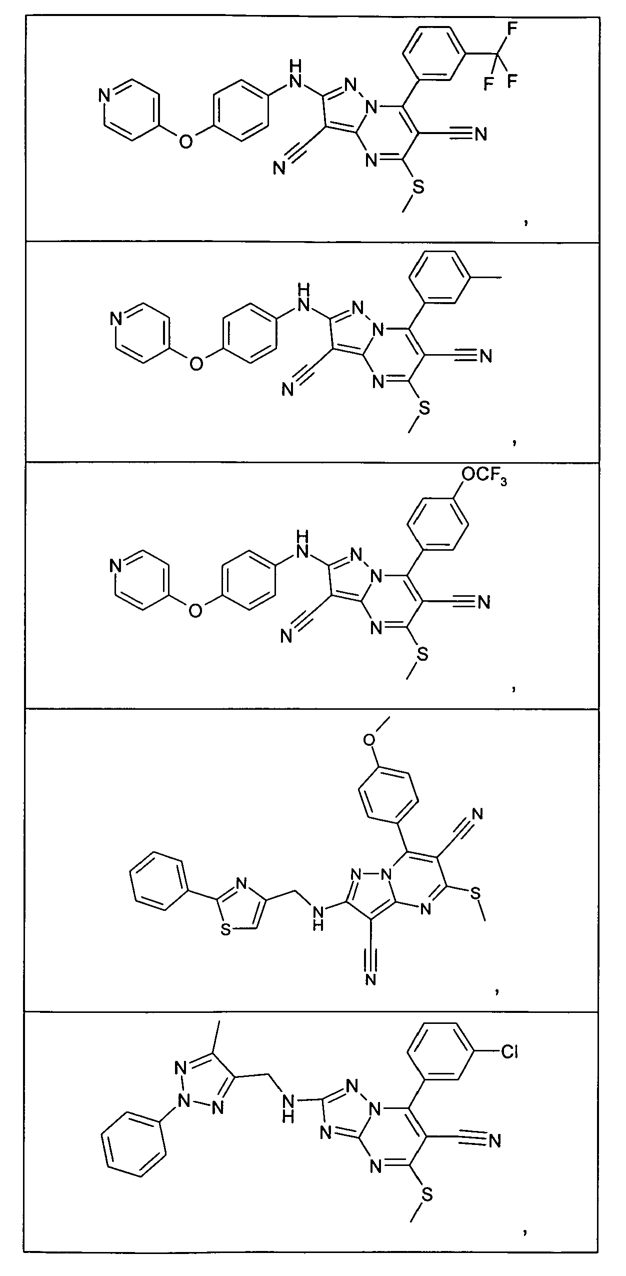 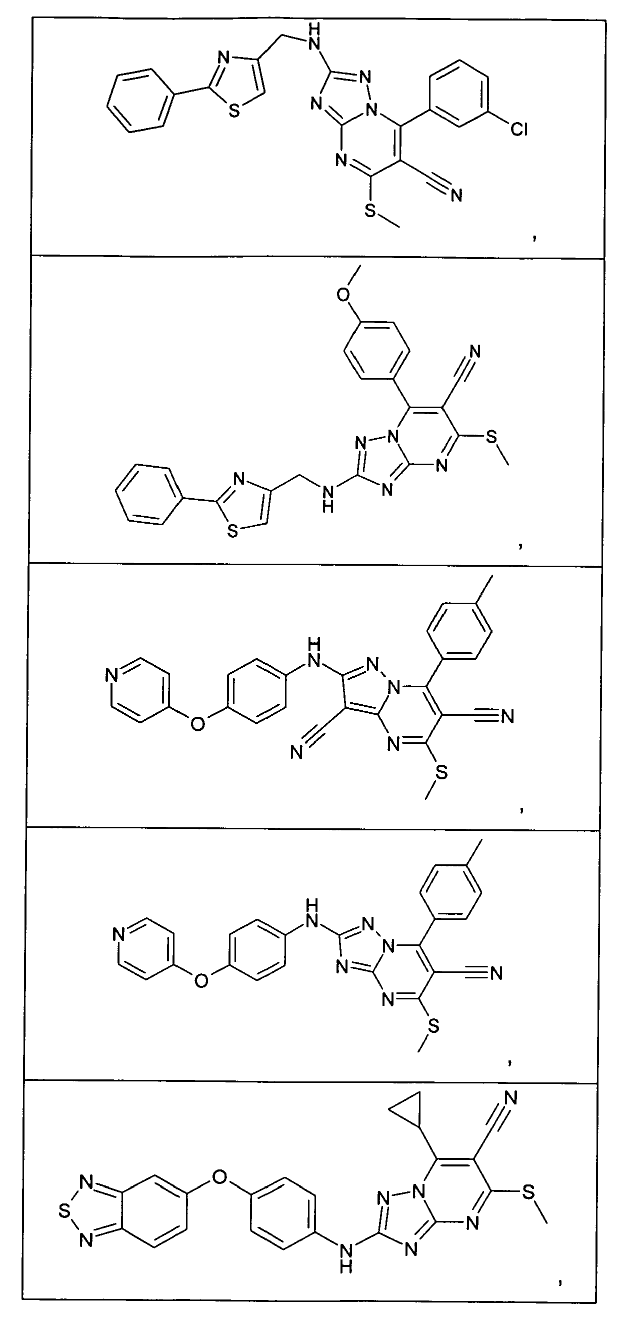 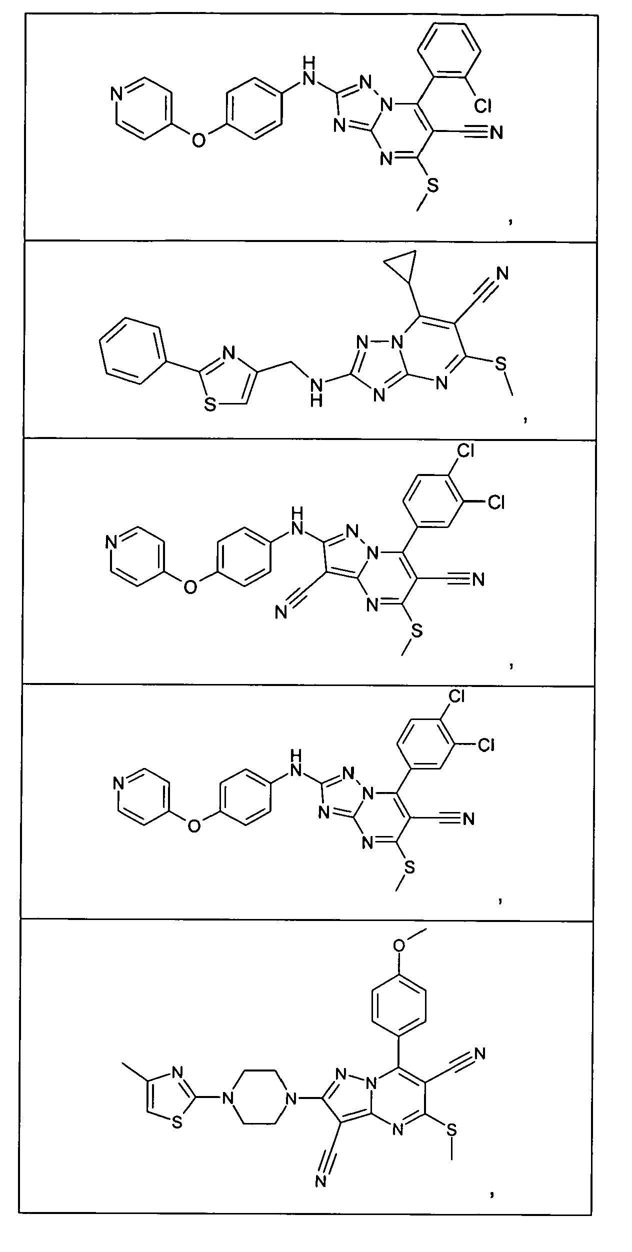 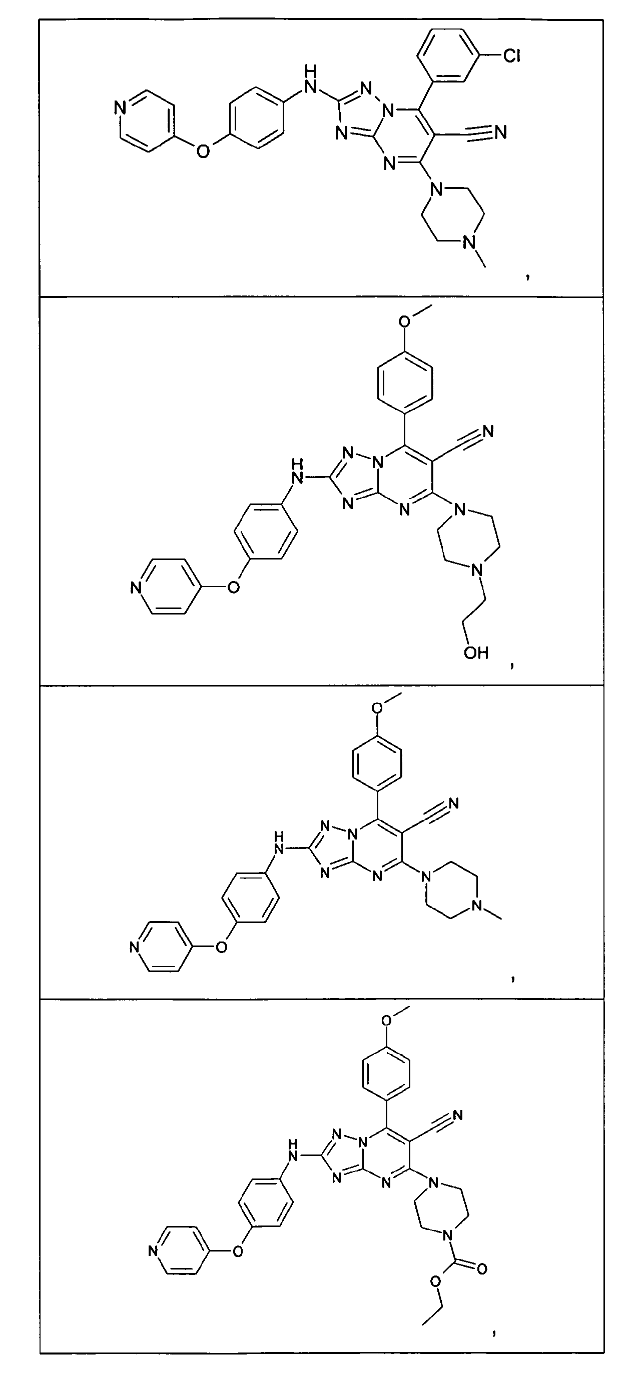 et les solvats, tautomères, sels et stéréoisomères pharmaceutiquement utilisables de ceux-ci, y compris des mélanges de ceux-ci en toutes proportions.

2. Médicaments comprenant au moins un composé selon la revendication 1 et/ou des sels, solvats, tautomères et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1 et de sels, solvats, tautomères et stéréoisomères pharmaceutiquement utilisables de ceux-ci, y compris des mélanges de ceux-ci en toutes proportions, pour la préparation d'un médicament destiné au traitement de maladies, où la maladie à traiter est une tumeur solide.

4. Utilisation selon la revendication 3, dans laquelle la tumeur solide provient du groupe des tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx et/ou des poumons.

5. Utilisation selon la revendication 3, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

6. Utilisation selon la revendication 3, dans laquelle la tumeur solide provient du groupe constitué par l'adénocarcinome du poumon, les carcinomes du poumon à petites cellules, le cancer du pancréas, les glioblastomes, le carcinome du côlon et le carcinome du sein.

7. Utilisation de composés selon la revendication 1 et de sels, solvats, tautomères et stéréoisomères pharmaceutiquement utilisables de ceux-ci, y compris des mélanges de ceux-ci en toutes proportions, pour la préparation d'un médicament destiné au traitement de maladies, où la maladie à traiter est une tumeur du système sanguin et immunitaire.

8. Utilisation selon la revendication 7, dans laquelle la tumeur provient du groupe constitué par la leucémie myéloïde aiguë, la leucémie myéloïde chronique, la leucémie lymphatique aiguë et/ou la leucémie lymphatique chronique.

9. Utilisation de composés selon la revendication 1 et de sels, solvats, tautomères et stéréoisomères pharmaceutiquement utilisables de ceux-ci, y compris des mélanges de ceux-ci en toutes proportions, pour la préparation d'un médicament destiné au traitement de maladies dans lesquelles l'angiogenèse est impliquée.

10. Utilisation selon la revendication 9, dans laquelle la maladie est une maladie oculaire.

11. Utilisation de composés selon la revendication 1 et de sels, solvats, tautomères et stéréoisomères pharmaceutiquement utilisables de ceux-ci, y compris des mélanges de ceux-ci en toutes proportions, pour la préparation d'un médicament destiné au traitement de maladies choisi parmi le group constitué par
la vascularisation rétinienne, la rétinopathie diabétique, la dégénérescence maculaire liée à l'âge, de maladies inflammatoires.

12. Utilisation selon la revendication 11, dans laquelle la maladie inflammatoire provient du groupe constitué par la polyarthrite rhumatoïde, le psoriasis, la dermite de contact et la réaction d'hypersensibilité retardée.

13. Utilisation de composés selon la revendication 1 et de sels, solvats, tautomères et stéréoisomères pharmaceutiquement utilisables de ceux-ci, y compris des mélanges de ceux-ci en toutes proportions, pour la préparation d'un médicament destiné au traitement de maladies choisi parmi le group constitué par l'ostéosarcome, l'arthrose, le rachitisme.

14. Utilisation de composés selon la revendication 1 et/ou de sels et de solvats physiologiquement acceptables de ceux-ci pour la préparation d'un médicament destiné au traitement de tumeurs solides, dans laquelle une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.

15. Utilisation de composés selon la revendication 1 et/ou de sels et de solvats physiologiquement acceptables de ceux-ci pour la préparation d'un médicament destiné au traitement de tumeurs solides, dans laquelle une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.
